# EUROPEAN PATENT APPLICATION

(11) **EP 4 450 117 A1**
(43) Date of publication of application: **23.10.2024**
(21) Application number: 22916157.5
(22) Date of filing: 28.12.2022
(51) Int. Cl.: A61M 39/26, A61M 39/06, A61M 39/22

(54) **ACCESS PORT MEMBER AND MANUFACTURING METHOD THEREOF**

(30) Priority: 28.12.2021 JP 2021215331; 28.12.2021 JP 2021215332; 28.12.2021 JP 2021215335
(71) Applicant: Nikkiso Company Limited, Tokyo 150-6022 (JP)
(72) Inventor: ONO, Kazuhide, Makinohara-shi, Shizuoka 421-0496 (JP); KAWAJIRI, Hiroyuki, Makinohara-shi, Shizuoka 421-0496 (JP); KATO, Ryo, Makinohara-shi, Shizuoka 421-0496 (JP)
(74) Representative: von Hirschhausen, Helge
(86) International application number: PCT/JP2022/048458
(87) International publication number: WO 2023/127935

(57) **Abstract**

Provided is an access port member capable of facilitating connection work of a connecting member while preventing a failure in closing of a slit. The access port member comprises: a body 8 to be connected to a predetermined portion of a blood circuit for causing a patient's blood to extracorporeally circulate, the body 8 internally including a flow path 8b for circulating the blood and having an opening 8c communicating with the flow path 8b; a valve 9 that is made of an elastic member covering the opening 8c and attached to an attachment part 8d of the body 8, the valve including a slit that makes a transition from a closed state to an open state when pressed by a tip 11a of a syringe 11 (connecting member) capable of collecting blood or injecting a drug; a cap part 10 fixed to the body 8 with the valve portion 9 interposed therebetween and the slit 9a facing outward; and a rib 9b formed integrally along the periphery of the slit 9a in the valve 9.

## Description

### Technical Field

The present invention relates to an access port member and a method of manufacturing the access port member which is to be connected to a blood circuit for causing a patient's blood to extracorporeally circulate to enable collection of blood or injection of a drug with a connecting member such as a syringe.

### Background Art

In general, a blood circuit for causing a patient's blood to extracorporeally circulate used in a blood purification treatment such as a hemodialysis treatment is mainly comprised of a flexible tube, and an access port member (may also be referred as a "rubber button" or "coinfusion member") is connected to a predetermined portion of the blood circuit. For example, as disclosed in PTL 1, a known access port member may be mentioned, which includes: a body connected to a blood circuit; a valve in which a slit is formed; and a cap part fixed to the body with the valve interposed therebetween.

Because the known access port member is connected to a blood circuit, blood can be collected (blood collection) into a syringe or a liquid drug can be injected (drug injection) from a syringe by pressing the peripheral portion of the slit in the valve with the tip of a connecting member such as a syringe to cause a transition of the slit from a closed state to an open state by a pressing force. Since the valve is made of an elastic member such as a rubber material, the closed state of the slit can be maintained by the elastic force.

Furthermore, the cap part in the known access port member has a locking part with a thread shape to be screwed and locked into a thread shape formed in the connecting member such as a syringe. The tip of a syringe or the like is pressed against the front surface of the valve to cause a transition of the slit to an open state, then the thread shape of the locking part is screwed and locked into the thread shape of the syringe or the like, thus the syringe or the like can be connected and held at the access port member.

### Citation List

### Patent Literature

PTL 1: Japanese Unexamined Patent Application Publication (Translation of PCT Application) No. 2006-501976

### Summary of Invention

### Technical Problem

However, when the valve of a known access port member is repeatedly pressed by the tip of a syringe or the like, a difference in level may occur in the slit, which may cause a failure in closing. Measures may be taken such as making the entire valve thicker so that a difference in level is unlikely to occur in the slit; however, in this case, a large pressing force is necessary to achieve an open state of the slit, thus a connection work for a syringe or the like may not be easily performed.

In the known access port member, when the tip of a syringe or the like causes a transition of the slit of the valve to an open state, the tapered surface formed on the cap part and the tapered surface formed on the syringe or the like are brought into contact to make sealing, thus if a dimension error occurs in the tapered surfaces, the slit is opened with improper sealing, so that liquid may be leaked to the outside.

In the known access port member, if a dimension error occurs in the thread shape of the locking part, when the thread shape of the locking part is smaller than the set dimensions, screwing between the thread shape of the locking part and the thread shape of the syringe or the like becomes tight, and the workability of locking is reduced, whereas when the thread shape of the locking part is larger than the set dimensions, screwing between the thread shape of the locking part and the thread shape of the syringe or the like becomes loose, thus the connection state with the syringe or the like may not be securely and reliably locked.

The present invention has been conceived in view of the above circumstances and it is an object to provide an access port member capable of facilitating connection work of a connecting member while preventing a failure in closing of the slit, an access port member capable of avoiding leakage of liquid to the outside when the slit of the valve is in an open state by securing the sealing between the slit and the connecting member, and an access port member and a method of manufacturing the access port member which are capable of smoothly performing the lock work by the locking part as well as securely and reliably performing the locking by the locking part.

### Solution to Problem

An access port member according to an embodiment of the present invention includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; a cap part fixed to the body with the valve interposed therebetween and the slit facing outward, the cap part having a locking part with a thread shape to be screwed and locked into a thread shape formed in the connecting member; and a rib integrally formed over a periphery of the slit in the valve.

An access port member according to an embodiment of the present invention includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; a cap part fixed to the body with the valve interposed therebetween and the slit facing outward, the cap part having a locking part with a thread shape to be screwed and locked into a thread shape formed in the connecting member; and a seal part formed over a periphery of the slit in the valve and configured to, when the slit is in an open state, match and seal the tip of the connecting member.

In addition, an access port member according to an embodiment of the present invention includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; and a cap part fixed to the body with the valve interposed therebetween and the slit facing outward, the cap part having a locking part with a thread shape to be screwed and locked into a thread shape formed in the connecting member. The thread shape of the locking part is formed such that a thread height at a terminal end is higher than a thread height in other portions.

### Advantageous Effects of Invention

According to the present invention, an access port member includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; a cap part fixed to the body with the valve interposed therebetween and the slit facing outward, the cap part having a locking part with a thread shape to be screwed and locked into a thread shape formed in the connecting member; and a rib integrally formed over a periphery of the slit in the valve, thus connection work of the connecting member can be facilitated while preventing a failure in closing of the slit.

According to the present invention, an access port member includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; a cap part fixed to the body with the valve interposed therebetween and the slit facing outward, the cap part having a locking part with a thread shape to be screwed and locked into a thread shape formed in the connecting member; and a seal part formed over a periphery of the slit in the valve and configured to, when the slit is in an open state, match and seal the tip of the connecting member, thus when the slit of the valve is in an open state, leakage of liquid to the outside can be avoided by securing the sealing between the slit and the connecting member.

Furthermore, according to the present invention, an access port member includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; and a cap part fixed to the body with the valve interposed therebetween and the slit facing outward, the cap part having a locking part with a thread shape to be screwed and locked into a thread shape formed in the connecting member. The thread shape of the locking part is formed such that a thread height at a terminal end is higher than a thread height in other portions, thus lock work by the locking part can be smoothly performed, and locking by the locking part can be securely and reliably conducted.

### Brief Description of Drawings

[Fig. 1] Fig. 1 schematically illustrates a blood circuit to which an access port member according to an embodiment of the present invention is applied.
[Fig. 2] Fig. 2 is a perspective view illustrating the access port member.
[Fig. 3] Fig. 3 shows a plan view and a side view illustrating the access port member.
[Fig. 4] Fig. 4 is a section taken along line IV-IV given in Fig. 3.
[Fig. 5] Fig. 5 is a section taken along line V-V given in Fig. 3.
[Fig. 6] Fig. 6 is an exploded perspective view illustrating the access port member.
[Fig. 7] Fig. 7 is a plan view illustrating a body of the access port member.
[Fig. 8] Fig. 8 is a three-view drawing illustrating a valve of the access port member.
[Fig. 9] Fig. 9 is a three-view drawing illustrating a cap part of the access port member.
[Fig. 10] Fig. 10 is a perspective view of the cap seen from above.
[Fig. 11] Fig. 11 is a perspective view of the cap seen from below.
[Fig. 12] Fig. 12 is a perspective view illustrating a syringe (connecting member) applied to the access port member.
[Fig. 13] Fig. 13 is a vertical sectional view illustrating the syringe.
[Fig. 14] Fig. 14 is a schematic view illustrating a process of connecting the syringe to the access port.
[Fig. 15] Fig. 15 is a sectional schematic view illustrating a state in which the tip of the syringe is brought into contact with the valve of the access port member.
[Fig. 16] Fig. 16 is a sectional schematic view illustrating a state (a state in which the tip of the syringe is pressed against the tip surface of a rib in the valve) in which the tip of the syringe is pressed against the valve of the access port member.
[Fig. 17] Fig. 17 is a sectional schematic view illustrating a state (a state in which the tip of the syringe is pressed against the tip surface and lateral surface of the rib in the valve) in which the tip of the syringe is pressed against the valve of the access port member.
[Fig. 18] Fig. 18 is an external view illustrating an access port member according to another embodiment of the present invention.
[Fig. 19] Fig. 19 is a vertical sectional view illustrating the access port member according to another embodiment.
[Fig. 20] Fig. 20 is a sectional schematic view illustrating a positional relationship with a slit formed in the valve of the access port member.
[Fig. 21] Fig. 21 is a sectional schematic view illustrating a positional relationship with a slit formed in the valve of an access port member in another embodiment of the present invention.
[Fig. 22] Fig. 22 is a sectional view illustrating an access port member according to another embodiment of the present invention.
[Fig. 23] Fig. 23 is a perspective view illustrating an access port member according to still another embodiment of the present invention.
[Fig. 24] Fig. 24 shows a plan view and a front view illustrating the access port member.
[Fig. 25] Fig. 25 is a section taken along line XXV-XXV given in Fig. 24.
[Fig. 26] Fig. 26 is a section taken along line XXVI-XXVI given in Fig. 24.
[Fig. 27] Fig. 27 is a three-view drawing illustrating a cap part in the access port member.
[Fig. 28] Fig. 28 is a section taken along line XXVIII-XXVIII given in Fig. 27.
[Fig. 29] Fig. 29 is a perspective view seen from above of a cap of an access port member according to a third embodiment.
[Fig. 30] Fig. 30 is a section for explaining a locking part (thread shape) of the cap part in the access port member.
[Fig. 31] Fig. 31 is a sectional schematic view illustrating a mold for manufacturing the cap part in the access port member.

### Description of Embodiments

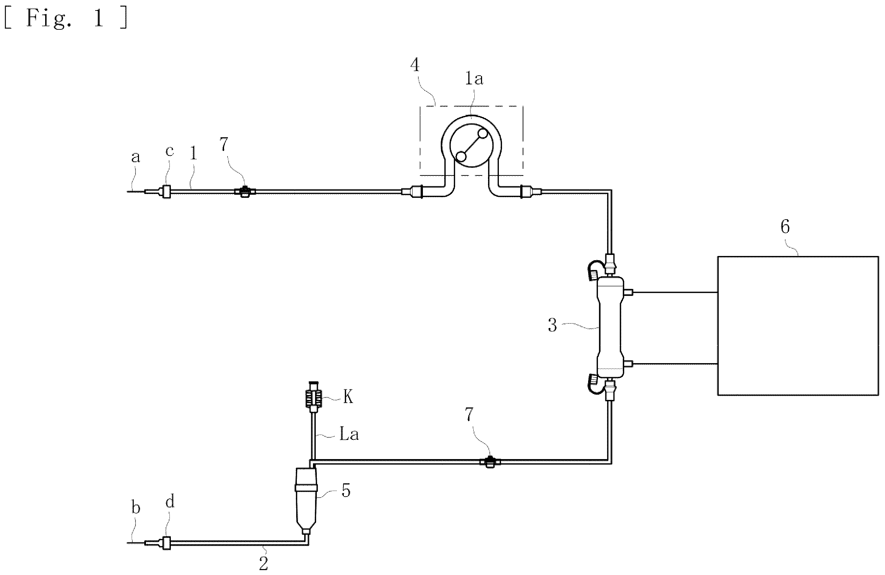

Hereinafter, an embodiment of the present invention will now be described specifically with reference to the drawings. An access port member according to a first embodiment is connected to a blood circuit for causing a patient's blood to extracorporeally circulate to enable a connecting member such as a syringe to collect blood or inject a drug, and as illustrated in Fig. 1, the blood circuit used in the present embodiment includes: a blood circuit having an arterial blood circuit 1 and a venous blood circuit 2; a dialyzer 3 as a blood purifier; a blood pump 4; and an air-trap chamber 5.

The arterial blood circuit 1 is formed of flexible tubes through which predetermined liquid is allowed to flow. The arterial blood circuit 1 is provided at the distal end thereof with a connector c, to which an arterial puncture needle a is attachable. The arterial blood circuit 1 is further provided with a squeezable tube 1a at a halfway position thereof. The squeezable tube 1a is attachable to the blood pump 4. The blood pump 4 is a peristaltic pump capable of delivering a priming solution or a patient's blood (liquid) in the blood circuit, and driving the blood pump 4 can cause a patient's blood to extracorporeally circulate.

The venous blood circuit 2 is formed of flexible tubes through which predetermined liquid is allowed to flow. The venous blood circuit 2 is provided at the distal end thereof with a connector d, to which a venous puncture needle b is attachable. The air-trap chamber 5 is connected to a halfway position of the venous blood circuit 2 to enable removal of the air contained in the blood that is under extracorporeal circulation in the blood circuit. A liquid drug infusion line La with a connecting part K attached to the distal end thereof for infusing liquid drug into the blood circuit extends from the top of the air-trap chamber 5. The blood circuit may not be provided with the liquid drug infusion line La at the top of the air-trap chamber 5.

The dialyzer 3 has, in a housing thereof, a plurality of hollow fibers each having microscopic holes (pores), and is capable of introducing dialysate from a dialysis device 6 as well as purifying the blood under extracorporeal circulation in the blood circuit by discharging drain liquid to the dialysis device 6. Specifically, the dialyzer 3 is connected between the arterial blood circuit 1 and the venous blood circuit 2, and the blood pump 4 is activated with the patient being punctured with the arterial puncture needle a and the venous puncture needle b, whereby the patient's blood is caused to extracorporeally circulate through the blood circuit (the arterial blood circuit 1 and the venous blood circuit 2) and the dialyzer 3 and is returned to the patient after being purified.

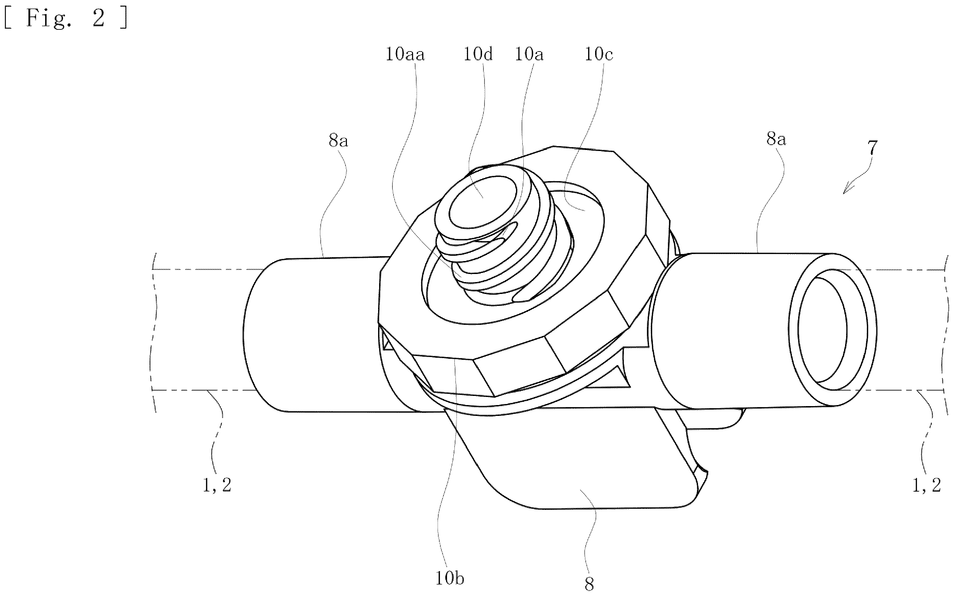

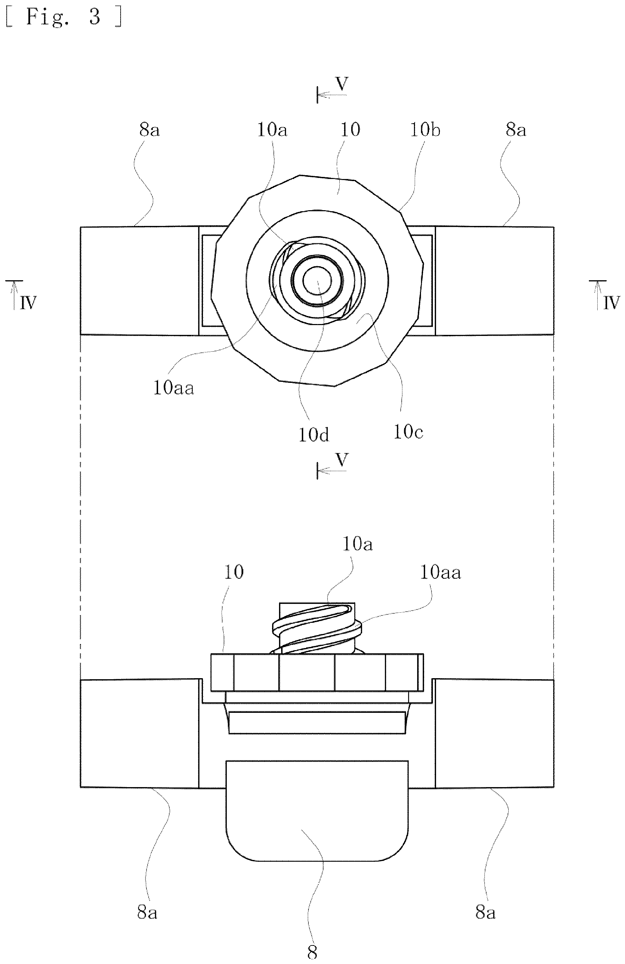

In the present embodiment, the access port member 7 is connected to a predetermined portion (for example, as illustrated, a portion between the connector c and the blood pump 4) of the arterial blood circuit 1, and a predetermined portion (for example, as illustrated, a portion between the dialyzer 3 and the air-trap chamber 5) of the venous blood circuit 2. The access port member 7 enables blood collection or drug injection for the blood that is under extracorporeal circulation in the blood circuit, and as illustrated in Figs. 2 to 6, includes a body 8, a valve 9, and a cap part 10. Note that the access port member 7 is not limited to the one connected to the blood circuit, and may be connected to, for example, the liquid drug infusion line La.

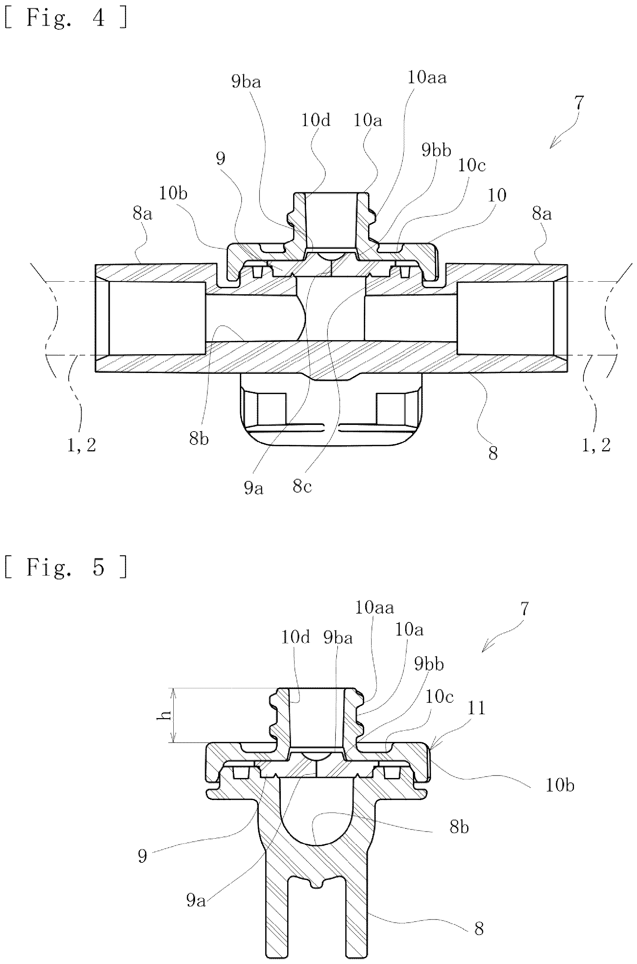

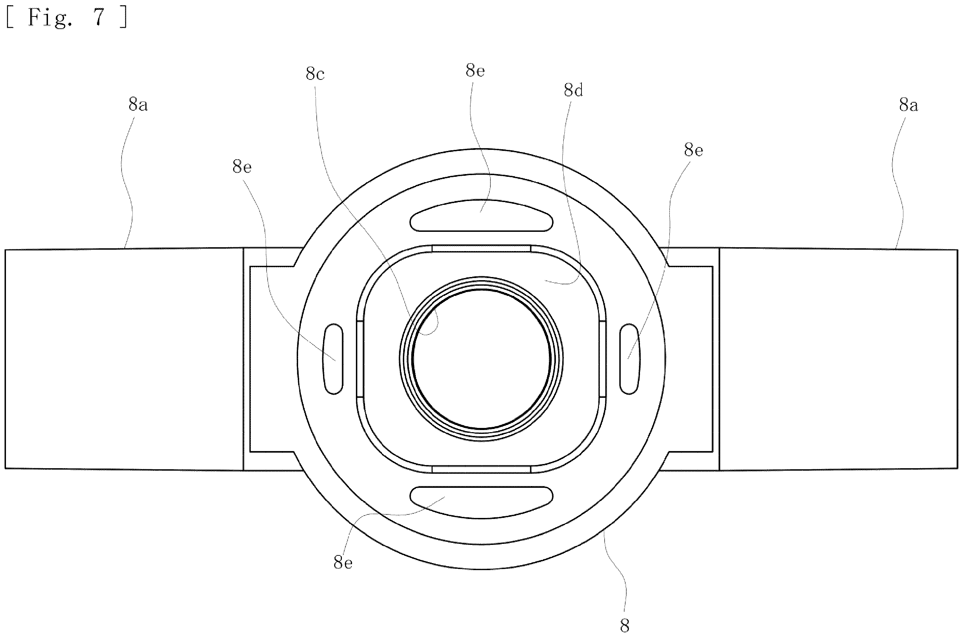

The body 8 is comprised of a resin molded part, and connected to a predetermined portion of the blood circuit (the arterial blood circuit 1 or the venous blood circuit 2) for causing a patient's blood to extracorporeally circulate, and as illustrated in Figs. 4 and 5, the body 8 internally includes a flow path 8b for circulating the blood, and an opening 8c communicating with the flow path 8b. As illustrated in Fig. 7, the body 8 includes: connecting portions 8a formed at both ends thereof and connectable to respective flexible tubes constituting the blood circuit; an attachment part 8d formed at the opening edge of the opening 8c allowing the valve 9 to be attached; and depressions 8e each serving as downgage formed around the attachment part 8d.

The attachment part 8d is in a depressed shape formed at the opening peripheral edge of the opening 8c, and has a shape copying the contour shape (shape copying a rectangle in the present embodiment) of the valve 9. The dimensions of the attachment part 8d are set to be slightly smaller than the external dimensions of the valve 9, allowing the valve 9 to be attached in a press-fitted state. Thus, the valve 9 is fixed with a compressive force applied inward from the outer peripheral edge, whereby the slit 9a is securely sealed.

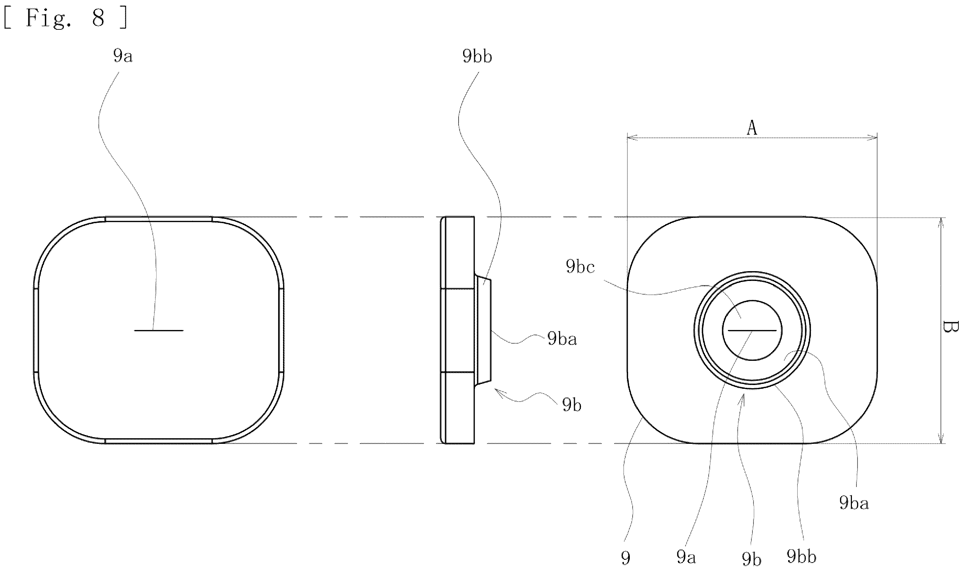

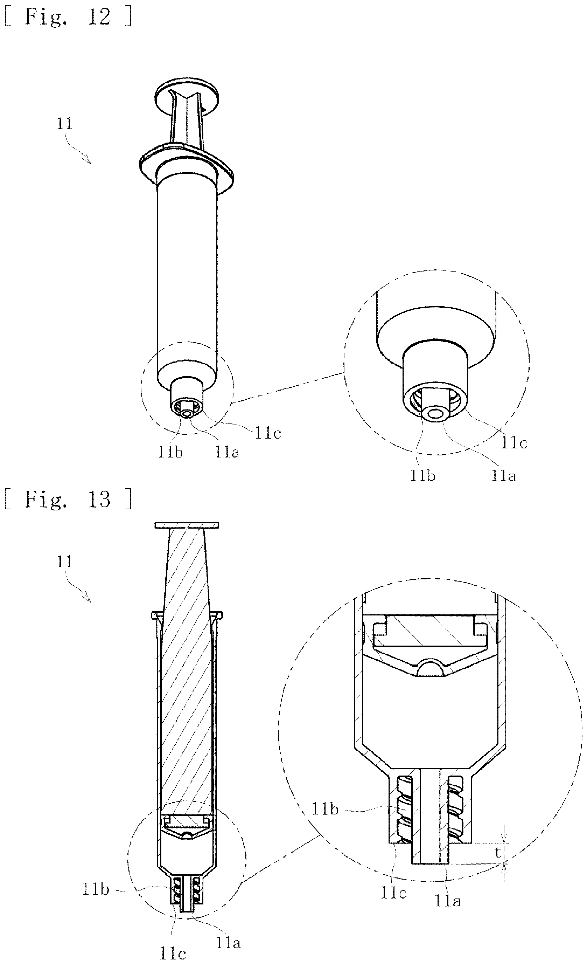

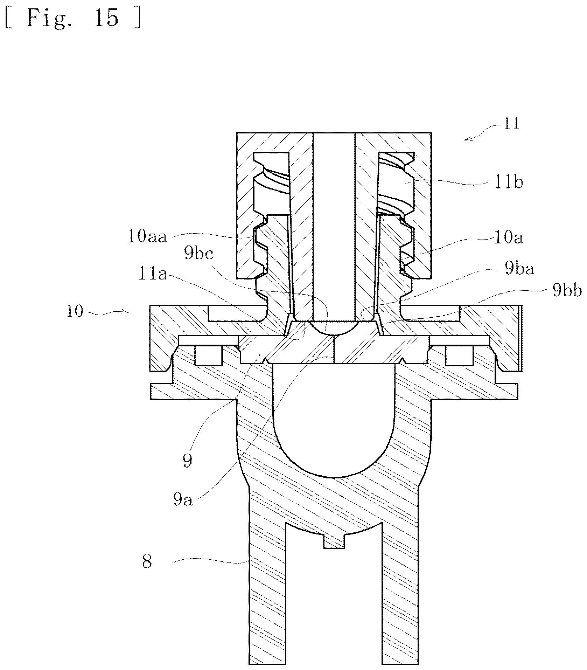

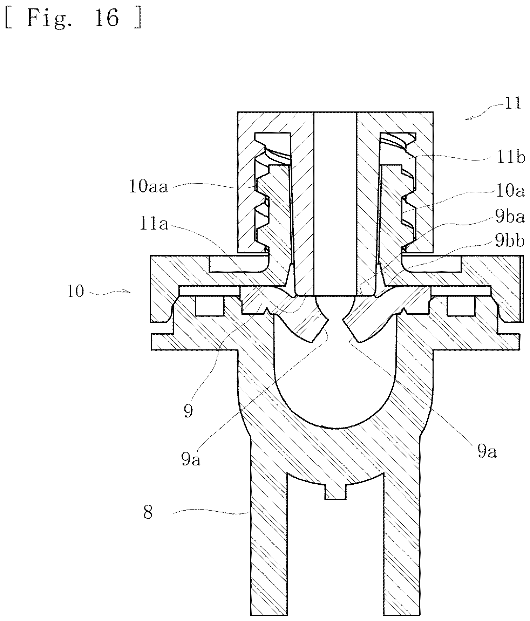

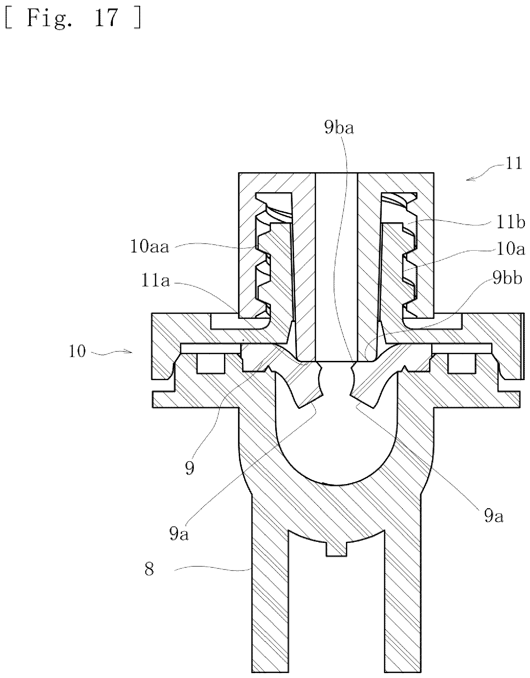

The valve 9 is made of an elastic member (rubber material) covering the opening 8c and attached to the attachment part 8d of the body 8, and the slit 9a in a line segment shape is formed at a central portion. As illustrated in Fig. 8, the valve 9 according to the present embodiment has the contour of a rectangle having a long side A and a short side B in a plan view, and the slit 9a extends in the direction parallel to the long side A of the rectangle. The slit 9a makes a transition from a closed state (see Fig. 15) to an open state (see Figs. 16 and 17) when the front surface of the valve 9 is pressed by a tip 11a (see Fig. 13) of a syringe 11 (connecting member) capable of collecting blood or injecting a drug.

As illustrated in Figs. 12 to 14, the syringe 11 used in the present embodiment is capable of sucking liquid or discharging liquid, specifically, capable of collecting blood (blood collection) from the tip 11a by a suction operation, or capable of injecting a drug (drug injection) from the tip 11a by a discharge operation. A thread shape 11b screwable into a thread shape 10aa formed on a locking part 10a of the cap part 10 is integrally formed on the outer peripheral portion of the tip 11a of the syringe 11.

The valve 9 according to the present embodiment includes an annular rib 9b that projects along the periphery of the slit 9a. The rib 9b having a tip surface 9ba and a lateral surface 9bb is composed of a protrusion (thick wall portion) surrounding the periphery of the slit 9a in the front surface of the valve 9 and continuously projecting, and is formed by copying the shape of the tip 11a of the syringe 11. As illustrated in Fig. 8, the rib 9b according to the present embodiment is circular in a plan view, and in an internal region 9bc thereof, the slit 9a in a line segment shape is formed. Thus, as illustrated in Figs. 15 to 17, the rib 9b is configured to, when the slit 9a makes a transition from a closed state to an open state, match the tip 11a of the syringe 11.

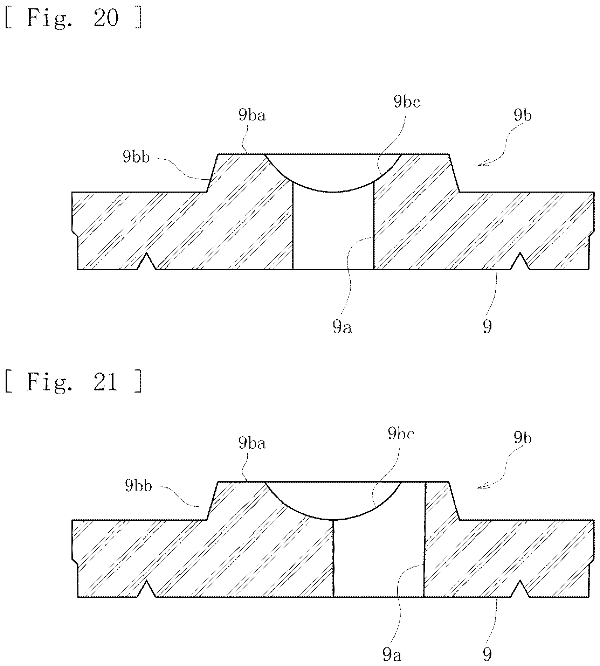

Specifically, as illustrated in Fig. 20, the internal region 9bc of the rib 9b is designed to be a thin wall portion, the slit 9a is formed in the thin wall portion, and the rib 9b is formed in the periphery of the thin wall portion, thereby increasing the intensity of the valve 9. Thus, since the slit 9a is formed in the thin wall portion of the internal region 9bc of the rib 9b, the slit 9a can make a transition from a closed state to an open state without applying a high pressing force to the rib 9b, and a sufficient intensity can be obtained by the rib 9b, thus even after repeated pressure to the rib 9b, a difference in level is prevented from occurring in the slit 9a. In addition to the case where the slit 9a is formed only in the internal region 9bc of the rib 9b as illustrated in Fig. 20, the slit 9a may be formed in the internal region 9bc, and part of the slit 9a may extend to the tip surface 9ba of the rib 9b as illustrated in Fig. 21.

Furthermore, as illustrated in Figs. 15 to 17, the rib 9b according to the present embodiment is configured to, when the slit 9a is in an open state, match and seal the tip 11a of the syringe 11 (connecting member), and in a process of insertion of the tip 11a of the syringe 11, during a transition of the slit 9a from a closed state (see Fig. 15) to an open state (see Figs. 16 and 17), and in a process of removal of the tip 11a of the syringe 11, during a transition of the slit 9a from an open state to a closed state, maintain a contact state with the tip 11a of the syringe 11.

Specifically, when the slit 9a is in a closed state, the tip 11a of the syringe 11 is brought into contact with the tip surface 9ba of the rib 9b (Fig. 15), and the syringe 11 is pushed in, thus the slit 9a makes a transition to an open state while the contact between the tip 11a of the syringe 11 and the tip surface 9ba of the rib 9b is being maintained (Fig. 16). When the syringe 11 is further pushed in, the tip 11a of the syringe 11 is brought into contact with the lateral surface 9bb subsequent to contact with the tip surface 9ba of the rib 9b (Fig. 17), thus sealing between the tip 11a of the syringe 11 and the rib 9b is maintained through the state illustrated in Figs. 15 to 17. However, even when a length t of projection (see Fig. 13) from the terminal of the thread shape 11b at the tip 11a of the syringe 11 is small, and the syringe 11 cannot be pushed in as shown in Fig. 17, the slit 9a can assume an open state while the contact between the tip 11a of the syringe 11 and the tip surface 9ba of the rib 9b being maintained as illustrated in Fig. 16. In replacement of or in addition to the manner in which the tip 11a of the syringe 11 comes into contact with the tip surface 9ba of the rib 9b to maintain the sealing in this way, the tip 11a of the syringe 11 may come into contact with the lateral surface 9bb of the rib 9b to maintain the sealing.

Although the rib 9b according to the present embodiment is composed of the protrusion formed on the front surface of the valve 9, the rib 9b may be composed of the protrusion formed on the rear surface of the valve 9, or may be composed of the protrusion formed on both front and rear surfaces. Although the rib 9b according to the present embodiment is circular in a plan view, the rib 9b may be elliptical or rectangular in a plan view, and the slit 9a may be formed in the internal region 9bc. In addition to the manner in which the slit 9a is formed in a line segment shape, the slit 9a may be formed in a cross shape.

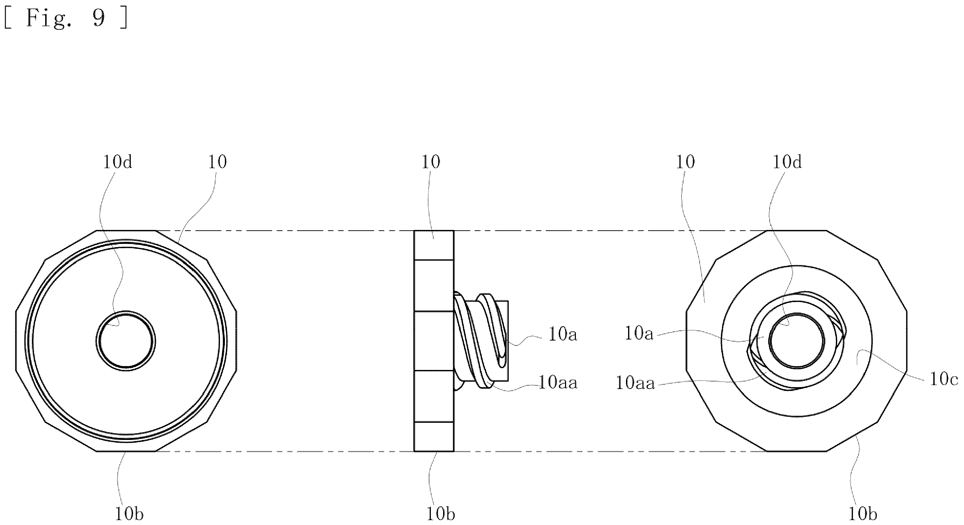

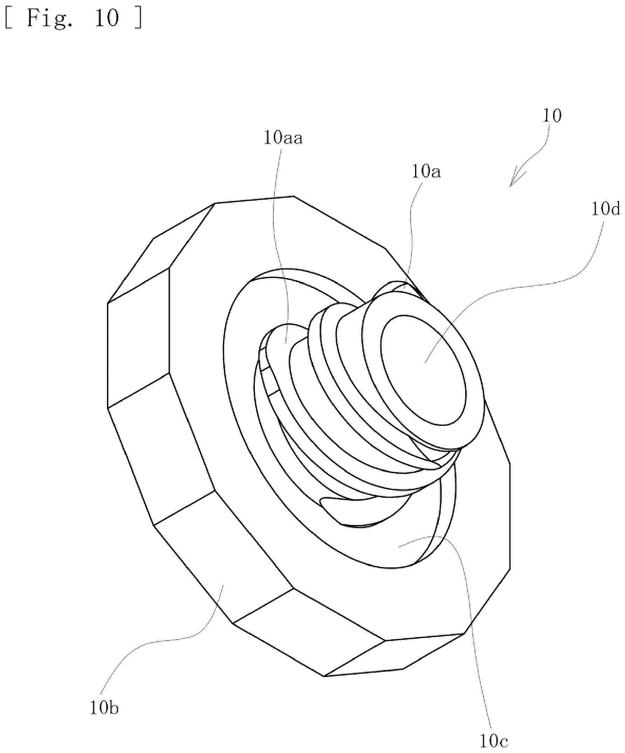

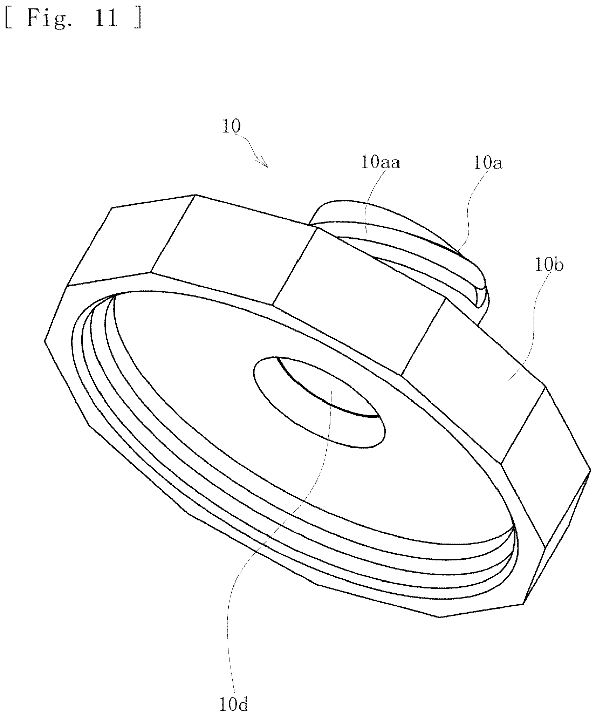

The cap part 10 is comprised of a component obtained by resin molding, and as illustrated in Figs. 4 and 5, is welded and fixed to the body 8 with the valve 9 interposed therebetween and the slit 9a facing outward. As illustrated in Figs. 9 to 11, the cap part 10 includes a locking part 10a, an outer peripheral edge 10b, an inner peripheral depression 10c, and a communication hole 10d. Specifically, the locking part 10a is formed to project along the central axis of the cap part 10, the inner peripheral depression 10c is formed around the locking part 10a, and the outer peripheral edge 10b having a large thickness is located on an outer diameter side of the inner peripheral depression 10c.

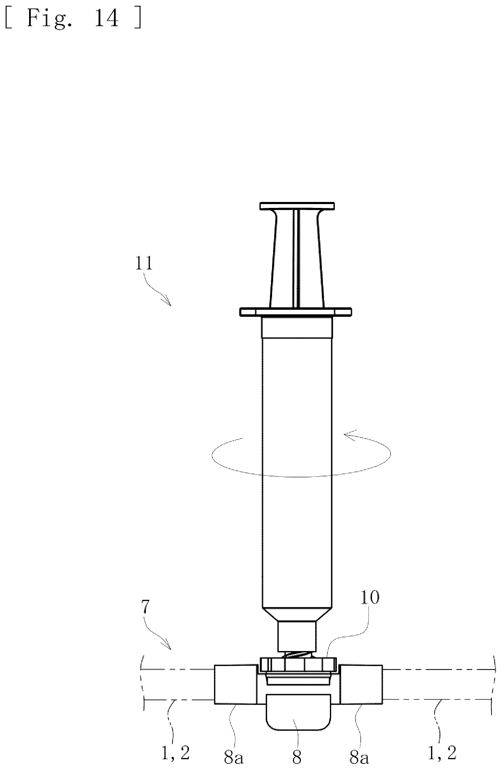

The locking part 10a internally includes the communication hole 10d through which the tip 11a of the syringe 11 is insertable and removable, and a spiral thread shape 10aa configured to match and screw into the thread shape 11b of the syringe 11 is integrally formed on the outer peripheral surface. Consequently, as illustrated in Fig. 14, the tip 11a of the syringe 11 is inserted into the communication hole 10d, and the syringe 11 is rotated, thus the thread shape 11b of the syringe 11 and the thread shape 10aa of the locking part 10a are screwed together and connected, whereby the syringe 11 can be locked (fixed) and connected to the access port member 7.

The access port member 7 according to the present embodiment includes: the body 8 having the opening 8c; the valve 9 made of an elastic member covering the opening 8c and attached to the attachment part 8d of the body 8, the valve 9 including the slit 9a that makes a transition from a closed state to an open state when pressed by the tip 11a of the syringe 11 (connecting member) capable of collecting blood or injecting a drug; the cap part 10 fixed to the body 8 with the valve 9 interposed therebetween and the slit 9a facing outward; and the rib 9b integrally formed over the periphery of the slit 9a in the valve 9, thus, the access port member 7 can facilitate connection work of the syringe 11 (connecting member) while preventing a failure in closing of the slit 9a.

In particular, the body 8 is connected to a predetermined portion of the blood circuit for causing a patient's blood to extracorporeally circulate, thus the following effects can be achieved. Specifically, occurrence of a stagnation point on the rear surface of the valve 9 may cause blood clotting, thus such an occurrence needs to be reduced as much as possible. However, when the valve 9 is located closer to the flow route to prevent stagnation, the valve 9 cannot be sufficiently pressed by the tip 11a of the syringe 11, which makes it difficult to open the slit 9a. Thus, providing the valve 9 with the rib 9b as in the present embodiment allows the valve 9 to be sufficiently pressed by the tip 11a of the syringe 11, whereby both easiness of opening the slit 9a and less occurrence of a stagnation point can be achieved.

The rib 9b according to the present embodiment is composed of a protrusion formed on the front surface, the rear surface, or both surfaces of the valve 9, is circular, elliptical or rectangular in a plan view, and includes the slit 9a in the internal region 9bc, thus elastic deformation is made easily by reducing an elastic force around the slit 9a so that an open state can be achieved, and a difference in level can be prevented from occurring by improving the restoring force after the elastic deformation with the rib 9b.

As illustrated in Fig. 20, the slit 9a is formed in the internal region 9bc of the rib 9b, and part of the slit 9a may extend to the tip surface 9ba of the rib 9b, thus the slit 9a can be formed in the valve 9 without requiring a high positioning accuracy. Furthermore, the slit 9a is formed in a line segment shape or a cross shape, thus an open state can be favorably achieved by pressing the tip 11a of the syringe 11.

In addition, the cap part 10 according to the present embodiment includes the locking part 10a with the thread shape 10aa to be screwed and locked into the thread shape 11b of the syringe 11 (connecting member), thus the following effects can be achieved. Specifically, since the dimensions of the locking part 10a are strictly defined by a standard, the slit 9a of the valve 9 may not be sufficiently pressed by the tip 11a of the syringe 11 (connecting member), thus the slit 9a may not be sufficiently opened. However, when the valve 9 is moved to the side where the syringe 11 is connected to prevent such a failure (for example, when the front surface of the valve 9 is superficialized to the side where the syringe 11 is connected), the valve 9 is made thicker, making it further difficult to open the slit 9a. When the valve 9 is made thinner and the valve 9 is superficialized, a liquid stagnation point occurs in greater number on the flow route side of the valve 9, and stagnation of blood and liquid drug is likely to occur, and in particular, when blood stagnates, blood clotting may occur. Thus, a structure needs to be created in which the slit 9a is reliably opened and stagnation is unlikely to occur. Thus, according to the present embodiment, providing the valve 9 with the rib 9b allows the tip 11a of the syringe 11 to sufficiently press the valve 9, whereby both easiness of opening the slit 9a and prevention of a stagnation point can be achieved.

The connecting member to be used is not limited to the syringe 11, and may be another connecting member (for example, a connecting member attached to the distal end of an infusion tube) which has a tip capable of pressing the rib 9b, and causes a transition of the slit 9a from a closed state to an open state by pressing to enable blood collection or drug injection. The depressions 8e may not be formed in the body 8. The valve 9 is not limited to the one having the contour of a rectangle in a plan view, and may be square, circular, elliptical or rectangular in a plan view.

In addition, the present invention is not limited to the one connected to a flow route for blood in the blood circuit, and may be connected to various flow routes (such as a liquid drug infusion line or a liquid drug delivery line serving as a flow route for a liquid drug) extending from the blood circuit. More specifically, in addition to the blood circuit for causing a patient's blood to extracorporeally circulate, the present invention is applicable to a circuit connected to a liquid delivery circuit, used for collection of blood flowing in the liquid delivery circuit, sampling of a liquid drug, and infusion of a liquid drug. For application to a liquid delivery circuit, an access port member may be used with one end thereof connected to an infusion bag and the other end connected to a connector coupled to an infusion line such as an indwelling needle or a blood circuit to enable drug injection and sampling, or an access port member may be used with connected to a liquid drug delivery circuit extended from a blood purifier such as the dialyzer 3 or various filters such as a blood adsorber, and a plasma separator to enable drug injection and sampling.

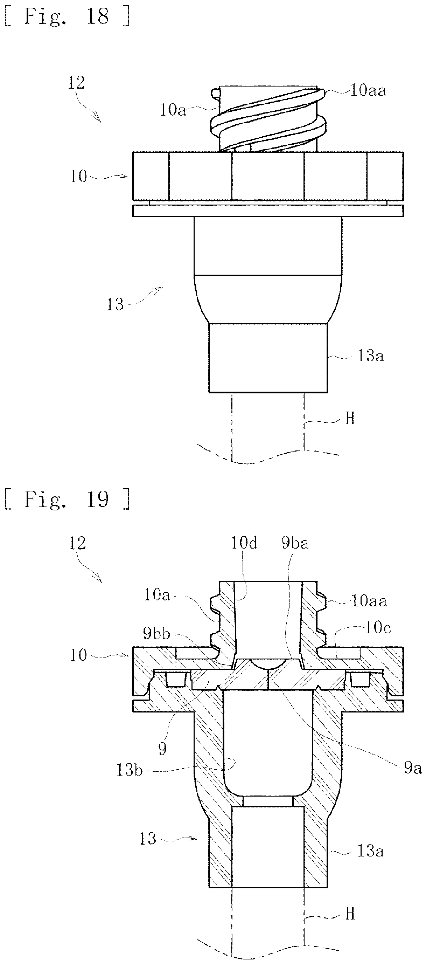

For application to a liquid delivery circuit, an access port member 12 having the configuration illustrated in Figs. 18 and 19 may be used. The access port member 12 includes: a body 13 having an opening 13b; a valve 9 made of an elastic member covering the opening 13b and attached to an attachment part of the body 13, the valve 9 including a slit 9a that makes a transition from a closed state to an open state when pressed by the tip 11a of the syringe 11 (connecting member) capable of collecting blood or injecting a drug; a cap part 10 fixed to the body 13 with the valve 9 interposed therebetween and the slit 9a facing outward, the cap part 10 having a locking part 10a with a thread shape 10aa to be screwed and locked into a thread shape 11b formed in the syringe 11; and a rib 9b integrally formed over the periphery of the slit 9a in the valve 9. The distal tip of a liquid delivery circuit H is to be connected to a connecting part 13a formed in the body 13. Note that the access port member 12 may be used instead of the connecting part K illustrated in Fig. 1.

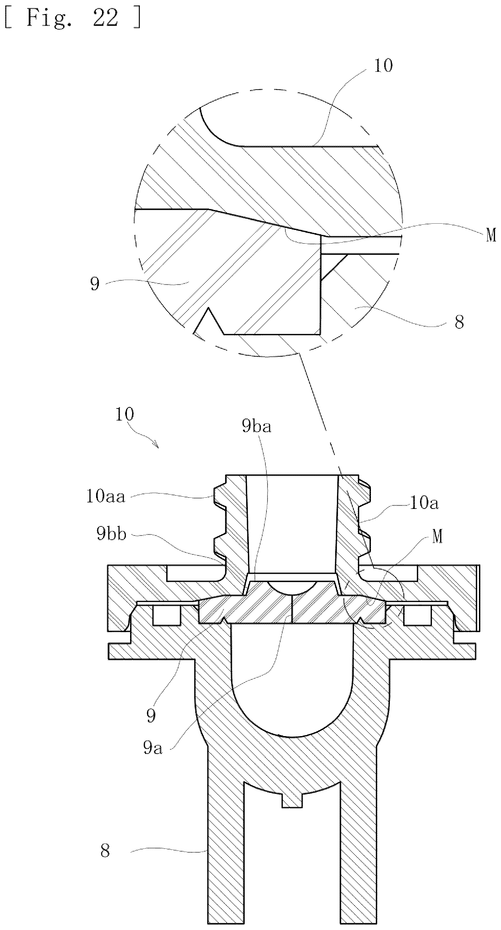

Meanwhile, when the valve 9 according to the embodiment is attached to the attachment part 8d of the body 8, the valve 9 is compressed in a direction in which the slit 9a is closed; however, as illustrated in Fig. 22, the valve may include the cap part 10 in which an inclined surface M extending parallel to the slit 9a is formed. In this case, the external dimensions of the valve 9 are set to be equal to the dimensions of the attachment part 8d, and after the valve 9 is attached to the attachment part 8d, the cap part 10 is fixed to the body 8, causing the inclined surface M to press against the valve 9, thus the valve 9 can be compressed in a direction in which the slit 9a is closed.

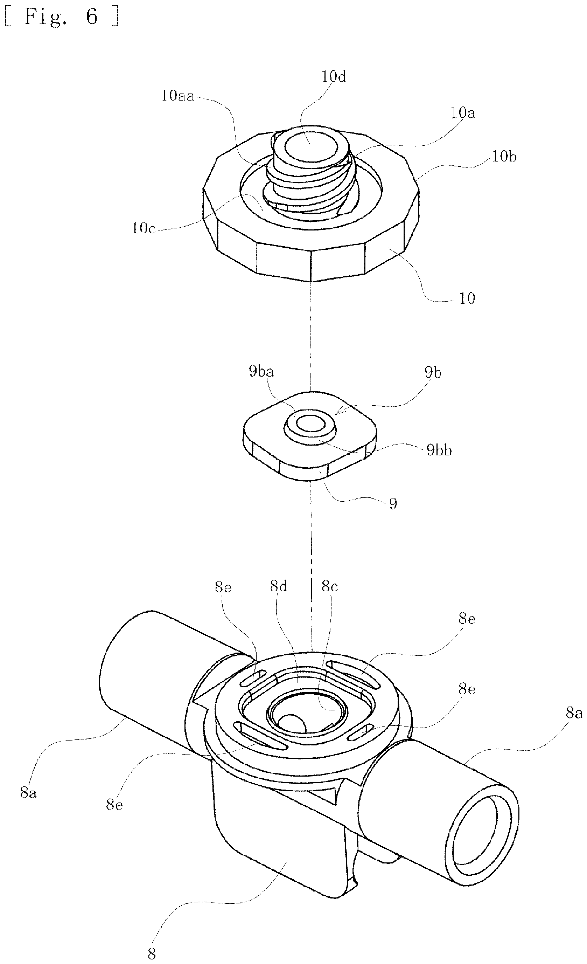

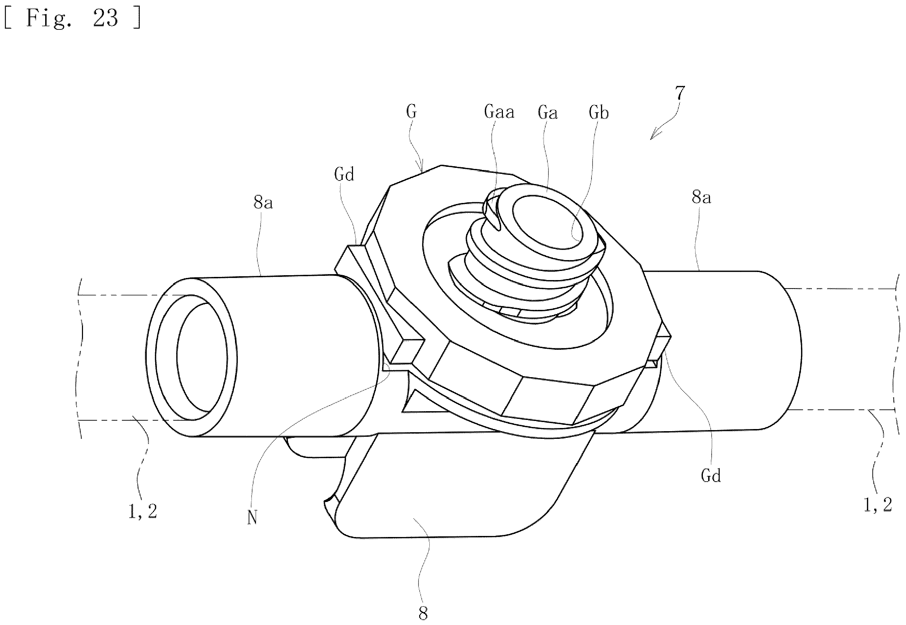

As illustrated in Figs. 23 to 26, the access port member 7 may include a cap part G. The access port member 7 includes the same body 8 as illustrated in Figs. 6 to 8, and the valve 9 in which the slit 9a is formed, and the connecting portions 8a are to be connected to a flow route in a blood circuit or the like. The cap part G in this case includes a locking part Ga fixed to the body 8 with the valve 9 interposed therebetween and the slit 9a facing outward in an insertion hole Gb, the locking part Ga with a thread shape Gaa to be screwed and locked into a thread shape formed in a connecting member such as the syringe 11. The same inclined surface M as previously described is formed on the rear surface of the cap part G, and after the valve 9 is attached to the attachment part 8d, the cap part G is fixed to the body 8, causing the inclined surface M to press against the valve 9, thus the valve 9 is compressed in a direction in which the slit 9a is closed.

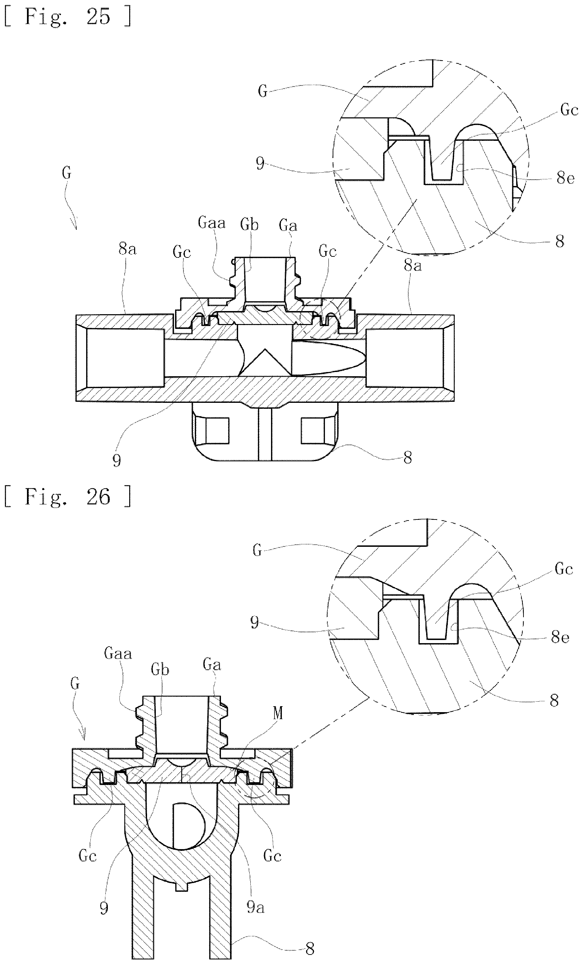

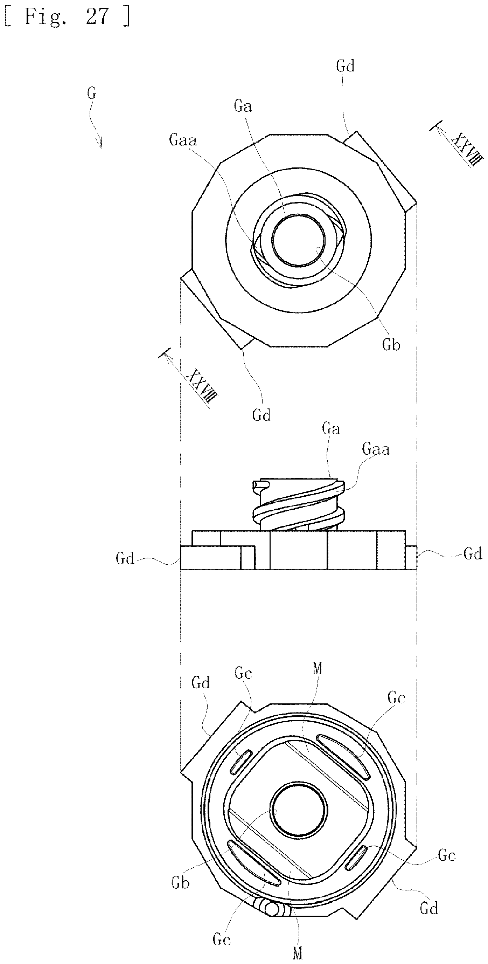

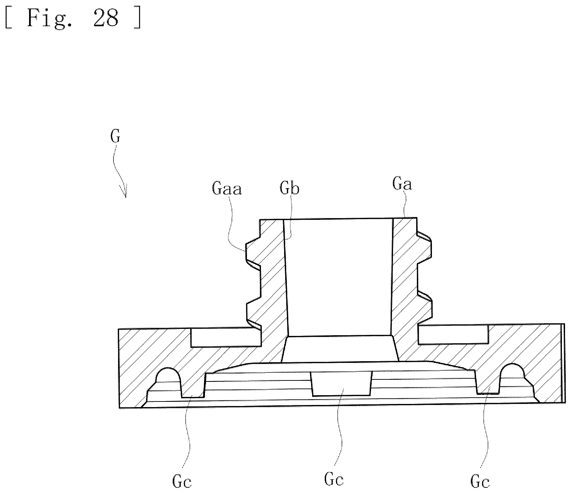

Furthermore, as illustrated in Figs. 27 and 28, in the cap part G, protrusions Gc formed at positions corresponding to depressions 8e of the body 8, and projection parts Gd formed at positions corresponding to notches N of the body 8 are integrally formed. As illustrated in Figs. 25 and 26, the protrusions Gc have a projecting shape which is to be fitted into the depressions 8e when the cap part G is welded and fixed to the body 8, and protrusions Gc at positions adjacent to the long sides A of the valve 9, and protrusions Gc at positions adjacent to the short sides B of the valve 9 are set to have different dimensions.

Thus, when the cap part G is attached to the attachment part 8d of the body 8, positioning in the rotational direction can be made by fitting the protrusions Gc into the depressions 8e. However, when the positioning in the rotational direction is not correct at the time of attachment of the cap part G, a protrusion Gc having a larger size cannot be fitted into a depression 8e having a smaller size, and comes into contact with its peripheral surface, thus energy more than necessary is consumed at the time of ultrasonic welding, which makes it possible to inform of an error. In particular, the cap part G includes the inclined surface M that causes the valve 9 to be compressed in a closing direction of the slit 9a, thus positioning can be accurately made so that the inclined surface M becomes parallel to the slit 9a.

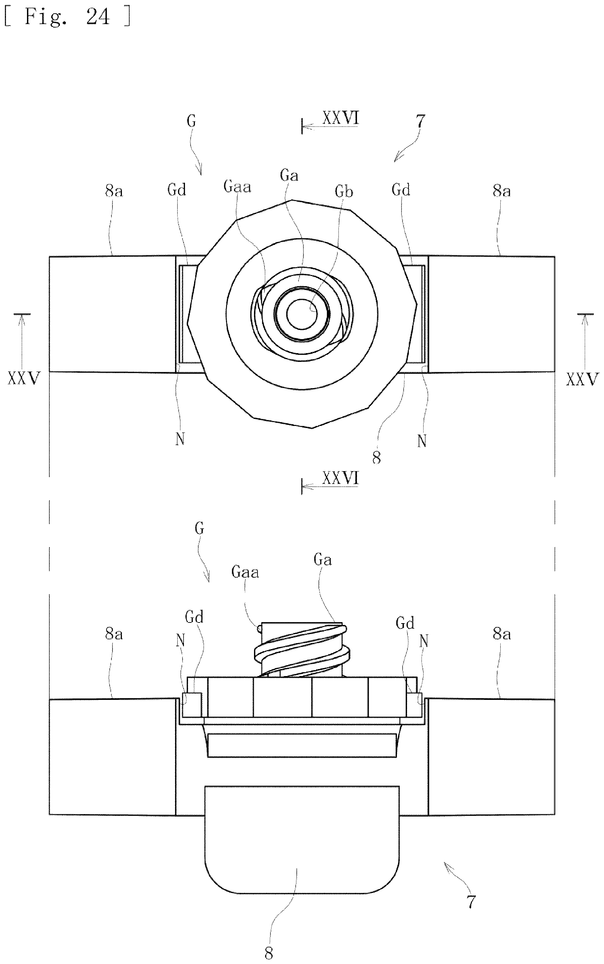

When the cap part G is welded and fixed to the body 8, if the positioning of the cap part G in the rotational direction is correct, each projection part Gd is opposed to the wall surface of a corresponding notch N as illustrated in Figs. 23 and 24. Thus, at the time of attachment of the cap part G, whether the positioning in the rotational direction is correct can be visually checked. As illustrated, the projection parts Gd are formed as a pair of right and left, but one of the pair may be formed, or alternatively, no projection part Gd may be formed.

Next, an access port member according to a second embodiment of the present invention will be described.

An access port member according to the second embodiment is connected to a blood circuit for causing a patient's blood to extracorporeally circulate to enable a connecting member such as a syringe to collect blood or inject a drug, and as illustrated in Fig. 1, the blood circuit used in the present embodiment includes: a blood circuit having an arterial blood circuit 1 and a venous blood circuit 2; a dialyzer 3 as a blood purifier; a blood pump 4; and an air-trap chamber 5. The blood circuit to be used is the same as in the first embodiment, thus a detailed description is omitted, and a description is given using the drawings in common with those of the first embodiment.

In the present embodiment, the access port member 7 is connected to a predetermined portion (for example, as illustrated, a portion between the connector c and the blood pump 4) of the arterial blood circuit 1, and a predetermined portion (for example, as illustrated, a portion between the dialyzer 3 and the air-trap chamber 5) of the venous blood circuit 2. The access port member 7 enables blood collection or drug injection for the blood that is under extracorporeal circulation in the blood circuit, and as illustrated in Figs. 2 to 6, includes the body 8, the valve 9, and the cap part 10. Note that the access port member 7 is not limited to the one connected to the blood circuit, and may be connected to, for example, the liquid drug infusion line La.

The body 8 is comprised of a resin molded part, and connected to a predetermined portion of the blood circuit (the arterial blood circuit 1 or the venous blood circuit 2) for causing a patient's blood to extracorporeally circulate, and as illustrated in Figs. 4 and 5, the body 8 internally includes a flow path 8b for circulating the blood, and an opening 8c communicating with the flow path 8b. As illustrated in Fig. 7, the body 8 includes: the connecting portions 8a formed at both ends thereof and connectable to respective flexible tubes constituting the blood circuit; the attachment part 8d formed at the opening edge of the opening 8c allowing the valve 9 to be attached; and the depressions 8e each serving as downgage formed around the attachment part 8d.

The attachment part 8d is in a depressed shape formed at the opening peripheral edge of the opening 8c, and has a shape copying the contour shape (shape copying a rectangle in the present embodiment) of the valve 9. The dimensions of the attachment part 8d are set to be slightly smaller than the external dimensions of the valve 9, allowing the valve 9 to be attached in a press-fitted state. Thus, the valve 9 is fixed with a compressive force applied inward from the outer peripheral edge, whereby the slit 9a is securely sealed.

The valve 9 is made of an elastic member (rubber material) covering the opening 8c and attached to the attachment part 8d of the body 8, and the slit 9a in a line segment shape is formed at a central portion. As illustrated in Fig. 8, the valve 9 according to the present embodiment has the contour of a rectangle having a long side A and a short side B in a plan view, and the slit 9a extends in the direction parallel to the long side A of the rectangle. The slit 9a makes a transition from a closed state (see Fig. 15) to an open state (see Figs. 16 and 17) when the front surface of the valve 9 is pressed by the tip 11a (see Fig. 13) of the syringe 11 (connecting member) capable of collecting blood or injecting a drug.

As illustrated in Figs. 12 to 14, the syringe 11 used in the present embodiment is capable of sucking liquid or discharging liquid, specifically, capable of collecting blood (blood collection) from the tip 11a by a suction operation, or capable of injecting a drug (drug injection) from the tip 11a by a discharge operation. The thread shape 11b screwable into the thread shape 10aa formed on the locking part 10a of the cap part 10 is integrally formed on the outer peripheral portion of the tip 11a of the syringe 11.

The valve 9 according to the present embodiment includes the annular rib 9b that projects along the periphery of the slit 9a. The rib 9b having the tip surface 9ba and the lateral surface 9bb constitutes the seal part of the present invention, and is composed of the protrusion (thick wall portion) surrounding the periphery of the slit 9a in the front surface of the valve 9 and continuously projecting, and is formed by copying the shape of the tip 11a of the syringe 11. In particular, the rib 9b according to the present embodiment is configured to be able to, when the slit 9a is in an open state, match and seal the tip 11a of the syringe 11.

Specifically, as illustrated in Figs. 15 to 17, the rib 9b according to the present embodiment is configured to, when the slit 9a is in an open state, match and seal the tip 11a of the syringe 11 (connecting member), and in a process of insertion of the tip 11a of the syringe 11, during a transition of the slit 9a from a closed state (see Fig. 15) to an open state (see Figs. 16 and 17), and in a process of removal of the tip 11a of the syringe 11, during a transition of the slit 9a from an open state to a closed state, maintain a contact state with the tip 11a of the syringe 11.

Specifically, when the slit 9a is in a closed state, the tip 11a of the syringe 11 is brought into contact with the tip surface 9ba of the rib 9b (Fig. 15), and the syringe 11 is pushed in, thus the slit 9a makes a transition to an open state while the contact between the tip 11a of the syringe 11 and the tip surface 9ba of the rib 9b is being maintained (Fig. 16). When the syringe 11 is further pushed in, the tip 11a of the syringe 11 is brought into contact with the lateral surface 9bb subsequent to contact with the tip surface 9ba of the rib 9b (Fig. 17), thus sealing between the tip 11a of the syringe 11 and the rib 9b is maintained through the state illustrated in Figs. 15 to 17. However, even when a length t of projection (see Fig. 13) from the terminal of the thread shape 11b at the tip 11a of the syringe 11 is small, and the syringe 11 cannot be pushed in as illustrated in Fig. 17, the slit 9a can assume an open state while the contact between the tip 11a of the syringe 11 and the tip surface 9ba of the rib 9b being maintained as illustrated in Fig. 16. In replacement of or in addition to the manner in which the tip 11a of the syringe 11 comes into contact with the tip surface 9ba of the rib 9b to maintain the sealing in this way, the tip 11a of the syringe 11 may come into contact with the lateral surface 9bb of the rib 9b to maintain the sealing.

As illustrated in Fig. 8, the rib 9b according to the present embodiment is circular in a plan view, and in the internal region 9bc thereof, the slit 9a in a line segment shape is formed. Thus, as illustrated in Figs. 15 to 17, the rib 9b is configured to, when the slit 9a makes a transition from a closed state to an open state, match the tip 11a of the syringe 11.

Specifically, as illustrated in Fig. 20, the internal region 9bc of the rib 9b is designed to be a thin wall portion, the slit 9a is formed in the thin wall portion, and the rib 9b is formed in the periphery of the thin wall portion, thereby increasing the intensity of the valve 9. Thus, since the slit 9a is formed in the thin wall portion of the internal region 9bc of the rib 9b, the slit 9a can make a transition from a closed state to an open state without applying a high pressing force to the rib 9b, and a sufficient intensity can be obtained by the rib 9b, thus even after repeated pressure to the rib 9b, a difference in level is prevented from occurring in the slit 9a. In addition to the case where the slit 9a is formed only in the internal region 9bc of the rib 9b as illustrated in Fig. 20, the slit 9a may be formed in the internal region 9bc, and part of the slit 9a may extend to the tip surface 9ba of the rib 9b as illustrated in Fig. 21.

Although the rib 9b according to the present embodiment is composed of the protrusion formed on the front surface of the valve 9, the rib 9b may be composed of the protrusion formed on both front and rear surfaces. In this case, the rib formed on the front surface of the valve 9 constitutes the seal part that matches and seals the tip 11a of the syringe 11. In addition to the manner in which the slit 9a is formed in a line segment shape, the slit 9a may be formed in a cross shape.

The cap part 10 is comprised of a component obtained by resin molding, and as illustrated in Figs. 4 and 5, is welded and fixed to the body 8 with the valve 9 interposed therebetween and the slit 9a facing outward. As illustrated in Figs. 9 to 11, the cap part 10 includes the locking part 10a, the outer peripheral edge 10b, the inner peripheral depression 10c, and the communication hole 10d. Specifically, the locking part 10a is formed to project along the central axis of the cap part 10, the inner peripheral depression 10c is formed around the locking part 10a, and the outer peripheral edge 10b having a large thickness is located on an outer diameter side of the inner peripheral depression 10c.

The locking part 10a internally includes the communication hole 10d through which the tip 11a of the syringe 11 is insertable and removable, and the spiral thread shape 10aa configured to match and screw into the thread shape 11b of the syringe 11 is integrally formed on the outer peripheral surface. Consequently, as illustrated in Fig. 14, the tip 11a of the syringe 11 is inserted into the communication hole 10d, and the syringe 11 is rotated, thus the thread shape 11b of the syringe 11 and the thread shape 10aa of the locking part 10a are screwed together and connected, whereby the syringe 11 can be locked (fixed) and connected to the access port member 7.

The access port member 7 according to the present embodiment includes: the body 8 having the opening 8c; the valve 9 made of an elastic member covering the opening 8c and attached to the attachment part 8d of the body 8, the valve 9 including the slit 9a that makes a transition from a closed state to an open state when pressed by the tip 11a of the syringe 11 (connecting member) capable of collecting blood or injecting a drug; the cap part 10 fixed to the body 8 with the valve 9 interposed therebetween and the slit 9a facing outward; and the rib 9b as a seal part formed over the periphery of the slit 9a in the valve 9 and configured to, when the slit 9a is in an open state, match and seal the tip 11a of the syringe 11 (connecting member), thus when the slit 9a of the valve 9 is in an open state, leakage of liquid to the outside can be avoided by securing the sealing between the slit 9a and the syringe 11.

In particular, the body 8 is connected to a predetermined portion of the blood circuit for causing a patient's blood to extracorporeally circulate, thus the following effects can be achieved. Specifically, occurrence of a stagnation point on the rear surface of the valve 9 may cause blood clotting, thus such an occurrence needs to be reduced as much as possible. However, when the valve 9 is located closer to the flow route to prevent stagnation, the valve 9 cannot be sufficiently pressed by the tip 11a of the syringe 11, which makes it difficult to open the slit 9a. Thus, providing the valve 9 with the rib 9b as in the present embodiment allows the valve 9 to be sufficiently pressed by the tip 11a of the syringe 11, whereby both easiness of opening the slit 9a and less occurrence of a stagnation point can be achieved.

The rib 9b as a seal part maintains a contact state with the tip 11a of the syringe 11 (connecting member) during a transition of the slit 9a from a closed state to an open state and during a transition of the slit 9a from an open state to a closed state, thus in both cases where the syringe 11 is brought into contact with the front surface of the valve 9 to open the slit 9a and where the syringe 11 is moved away from the front surface of the valve 9 to close the slit 9a, leakage of liquid to the outside can be avoided by securing the sealing between the slit 9a and the syringe 11.

The seal part is comprised of the rib 9b which is formed by copying the shape of the tip 11a of the syringe 11 (connecting member), thus can avoid leakage of liquid to the outside by securing the sealing between the slit 9a and the syringe 11, and can facilitate connection work of the syringe 11 (connecting member) while preventing a failure in closing of the slit 9a.

As illustrated in Fig. 20, the slit 9a is formed in the internal region 9bc of the rib 9b, and part of the slit 9a may extend to the tip surface 9ba of the rib 9b, thus the slit 9a can be formed in the valve 9 without requiring a high positioning accuracy. Furthermore, the slit 9a is formed in a line segment shape or a cross shape, thus an open state can be favorably achieved by pressing the tip 11a of the syringe 11.

In addition, the cap part 10 according to the present embodiment includes the locking part 10a with the thread shape 10aa to be screwed and locked into the thread shape 11b of the syringe 11 (connecting member), thus the following effects can be achieved. Specifically, since the dimensions of the locking part 10a are strictly defined by a standard, the slit 9a of the valve 9 may not be sufficiently pressed by the tip 11a of the syringe 11 (connecting member), thus the slit 9a may not be sufficiently opened. However, when the valve 9 is moved to the side where the syringe 11 is connected to prevent such a failure (for example, when the front surface of the valve 9 is superficialized to the side where the syringe 11 is connected), the valve 9 is made thicker, making it further difficult to open the slit 9a. When the valve 9 is made thinner and the valve 9 is superficialized, a liquid stagnation point occurs in greater number on the flow route side of the valve 9, and stagnation of blood and liquid drug is likely to occur, and in particular, when blood stagnates, blood clotting may occur. Thus, a structure needs to be created in which the slit 9a is reliably opened and stagnation is unlikely to occur. Thus, according to the present embodiment, providing the valve 9 with the rib 9b allows the tip 11a of the syringe 11 to sufficiently press the valve 9, whereby both easiness of opening the slit 9a and prevention of a stagnation point can be achieved.

Although the present embodiment has been described above, the present invention is not limited to this, and the connecting member to be used is not limited to the syringe 11, and may be another connecting member (for example, a connecting member attached to the distal end of an infusion tube) which has a tip capable of pressing the rib 9b, and causes a transition of the slit 9a from a closed state to an open state by pressing to enable blood collection or drug injection. The depressions 8e may not be formed in the body 8. The valve 9 is not limited to the one having the contour of a rectangle in a plan view, and may be square, circular, elliptical or rectangular in a plan view.

In addition, the present invention is not limited to the one connected to a flow route for blood in the blood circuit, and may be connected to various flow routes (such as a liquid drug infusion line or a liquid drug delivery line serving as a flow route for a liquid drug) extending from the blood circuit. More specifically, in addition to the blood circuit for causing a patient's blood to extracorporeally circulate, the present invention is applicable to a circuit connected to a liquid delivery circuit, used for collection of blood flowing in the liquid delivery circuit, sampling of a liquid drug, and infusion of a liquid drug. For application to a liquid delivery circuit, an access port member may be used with one end thereof connected to an infusion bag and the other end connected to a connector coupled to an infusion line such as an indwelling needle or a blood circuit to enable drug injection and sampling, or an access port member may be used with connected to a liquid drug delivery circuit extended from a blood purifier such as the dialyzer 3 or various filters such as a blood adsorber, and a plasma separator to enable drug injection and sampling.

For application to a liquid delivery circuit, the access port member 12 having the configuration illustrated in Figs. 18 and 19 may be used. The access port member 12 includes: a body 13 having an opening 13b; a valve 9 made of an elastic member covering the opening 13b and attached to an attachment part of the body 13, the valve 9 including a slit 9a that makes a transition from a closed state to an open state when pressed by the tip 11a of the syringe 11 (connecting member) capable of collecting blood or injecting a drug; a cap part 10 fixed to the body 13 with the valve 9 interposed therebetween and the slit 9a facing outward, the cap part 10 having a locking part 10a with a thread shape 10aa to be screwed and locked into a thread shape 11b formed in the syringe 11; and a rib 9b integrally formed over the periphery of the slit 9a in the valve 9. The distal tip of a liquid delivery circuit H is to be connected to the connecting part 13a formed in the body 13. Note that the access port member 12 may be used instead of the connecting part K illustrated in Fig. 1.

Next, an access port member according to a third embodiment of the present invention will be described.

An access port member according to the third embodiment is connected to a blood circuit for causing a patient's blood to extracorporeally circulate to enable a connecting member such as a syringe to collect blood or inject a drug, and as illustrated in Fig. 1, the blood circuit used in the present embodiment includes: a blood circuit having an arterial blood circuit 1 and a venous blood circuit 2; a dialyzer 3 as a blood purifier; a blood pump 4; and an air-trap chamber 5. The blood circuit to be used is the same as in the first embodiment, thus a detailed description is omitted, and a description is given using the drawings in common with those of the first embodiment.

In the present embodiment, the access port member 7 is connected to a predetermined portion (for example, as illustrated, a portion between the connector c and the blood pump 4) of the arterial blood circuit 1, and a predetermined portion (for example, as illustrated, a portion between the dialyzer 3 and the air-trap chamber 5) of the venous blood circuit 2. The access port member 7 enables blood collection or drug injection for the blood that is under extracorporeal circulation in the blood circuit, and as illustrated in Figs. 2 to 6, includes the body 8, the valve 9, and the cap part 10. Note that the access port member 7 is not limited to the one connected to the blood circuit, and may be connected to, for example, the liquid drug infusion line La.

The body 8 is comprised of a resin molded part, and connected to a predetermined portion of the blood circuit (the arterial blood circuit 1 or the venous blood circuit 2) for causing a patient's blood to extracorporeally circulate, and as illustrated in Figs. 4 and 5, the body 8 internally includes a flow path 8b for circulating the blood, and an opening 8c communicating with the flow path 8b. As illustrated in Fig. 7, the body 8 includes: the connecting portions 8a formed at both ends thereof and connectable to respective flexible tubes constituting the blood circuit; the attachment part 8d formed at the opening edge of the opening 8c allowing the valve 9 to be attached; and the depressions 8e each serving as downgage formed around the attachment part 8d.

The attachment part 8d is in a depressed shape formed at the opening peripheral edge of the opening 8c, and has a shape copying the contour shape (shape copying a rectangle in the present embodiment) of the valve 9. The dimensions of the attachment part 8d are set to be slightly smaller than the external dimensions of the valve 9, allowing the valve 9 to be attached in a press-fitted state. Thus, the valve 9 is fixed with a compressive force applied inward from the outer peripheral edge, whereby the slit 9a is securely sealed.

The valve 9 is made of an elastic member (rubber material) covering the opening 8c and attached to the attachment part 8d of the body 8, and the slit 9a in a line segment shape is formed at a central portion. As illustrated in Fig. 8, the valve 9 according to the present embodiment has the contour of a rectangle having a long side A and a short side B in a plan view, and the slit 9a extends in the direction parallel to the long side A of the rectangle. The slit 9a makes a transition from a closed state (see Fig. 15) to an open state (see Figs. 16 and 17) when the front surface of the valve 9 is pressed by the tip 11a (see Fig. 12) of the syringe 11 (connecting member) capable of collecting blood or injecting a drug.

As illustrated in Figs. 12 to 14, the syringe 11 used in the present embodiment is capable of sucking liquid or discharging liquid, specifically, capable of collecting blood (blood collection) from the tip 11a by a suction operation, or capable of injecting a drug (drug injection) from the tip 11a by a discharge operation. The thread shape 11b screwable into the thread shape 10aa formed on the locking part 10a of the cap part 10 is integrally formed on the outer peripheral portion of the tip 11a of the syringe 11.

The valve 9 according to the present embodiment includes the annular rib 9b that projects along the periphery of the slit 9a. The rib 9b having the tip surface 9ba and the lateral surface 9bb is composed of a protrusion (thick wall portion) surrounding the periphery of the slit 9a in the front surface of the valve 9 and continuously projecting, and is formed by copying the shape of the tip 11a of the syringe 11. As illustrated in Fig. 8, the rib 9b according to the present embodiment is circular in a plan view, and in the internal region 9bc thereof, the slit 9a in a line segment shape is formed. Thus, as illustrated in Figs. 15 to 17, the rib 9b is configured to, when the slit 9a makes a transition from a closed state to an open state, match the tip 11a of the syringe 11.

Specifically, as illustrated in Fig. 20, the internal region 9bc of the rib 9b is designed to be a thin wall portion, the slit 9a is formed in the thin wall portion, and the rib 9b is formed in the periphery of the thin wall portion, thereby increasing the intensity of the valve 9. Thus, since the slit 9a is formed in the thin wall portion of the internal region 9bc of the rib 9b, the slit 9a can make a transition from a closed state to an open state without applying a high pressing force to the rib 9b, and a sufficient intensity can be obtained by the rib 9b, thus even after repeated pressure to the rib 9b, a difference in level is prevented from occurring in the slit 9a.

Furthermore, as illustrated in Figs. 15 to 17, the rib 9b according to the present embodiment is configured to, when the slit 9a is in an open state, match and seal the tip 11a of the syringe 11 (connecting member), and in a process of insertion of the tip 11a of the syringe 11, during a transition of the slit 9a from a closed state (see Fig. 15) to an open state (see Figs. 16 and 17), and in a process of removal of the tip 11a of the syringe 11, during a transition of the slit 9a from an open state to a closed state, maintain a contact state with the tip 11a of the syringe 11.

Specifically, when the slit 9a is in a closed state, the tip 11a of the syringe 11 is brought into contact with the tip surface 9ba of the rib 9b (Fig. 15), and the syringe 11 is pushed in, thus the slit 9a makes a transition to an open state while the contact between the tip 11a of the syringe 11 and the tip surface 9ba of the rib 9b is being maintained (Fig. 16). When the syringe 11 is further pushed in, the tip 11a of the syringe 11 is brought into contact with the lateral surface 9bb subsequent to contact with the tip surface 9ba of the rib 9b (Fig. 17), thus sealing between the tip 11a of the syringe 11 and the rib 9b is maintained through the state illustrated in Figs. 15 to 17. However, even when a length t of projection (see Fig. 13) from the terminal of the thread shape 11b at the tip 11a of the syringe 11 is small, and the syringe 11 cannot be pushed in as illustrated in Fig. 17, the slit 9a can assume an open state while the contact between the tip 11a of the syringe 11 and the tip surface 9ba of the rib 9b being maintained as illustrated in Fig. 16. In replacement of or in addition to the manner in which the tip 11a of the syringe 11 comes into contact with the tip surface 9ba of the rib 9b to maintain the sealing in this way, the tip 11a of the syringe 11 may come into contact with the lateral surface 9bb of the rib 9b to maintain the sealing.

Although the rib 9b according to the present embodiment is composed of the protrusion formed on the front surface of the valve 9, the rib 9b may be composed of the protrusion formed on the rear surface of the valve 9, or may be composed of the protrusion formed on both front and rear surfaces. Although the rib 9b according to the present embodiment is circular in a plan view, the rib 9b may be elliptical or rectangular in a plan view, and the slit 9a may be formed in the internal region 9bc. In addition to the manner in which the slit 9a is formed in a line segment shape, the slit 9a may be formed in a cross shape.

The cap part 10 is comprised of a component obtained by resin molding, and as illustrated in Figs. 4 and 5, is welded and fixed to the body 8 with the valve 9 interposed therebetween and the slit 9a facing outward. As illustrated in Figs. 9 to 11, 29, the cap part 10 includes the locking part 10a, the outer peripheral edge 10b, the inner peripheral depression 10c, and the communication hole 10d. Specifically, the locking part 10a is formed to project along the central axis L of the cap part 10, the inner peripheral depression 10c is formed around the locking part 10a, and the outer peripheral edge 10b having a large thickness is located on an outer diameter side of the inner peripheral depression 10c.

The locking part 10a internally includes the communication hole 10d through which the tip 11a of the syringe 11 is insertable and removable, and the spiral thread shape 10aa configured to match and screw into the thread shape 11b of the syringe 11 is integrally formed on the outer peripheral surface. Consequently, as illustrated in Fig. 14, the tip 11a of the syringe 11 is inserted into the communication hole 10d, and the syringe 11 is rotated, thus the thread shape 11b of the syringe 11 and the thread shape 10aa of the locking part 10a are screwed together and connected, whereby the syringe 11 can be locked (fixed) and connected to the access port member 7.

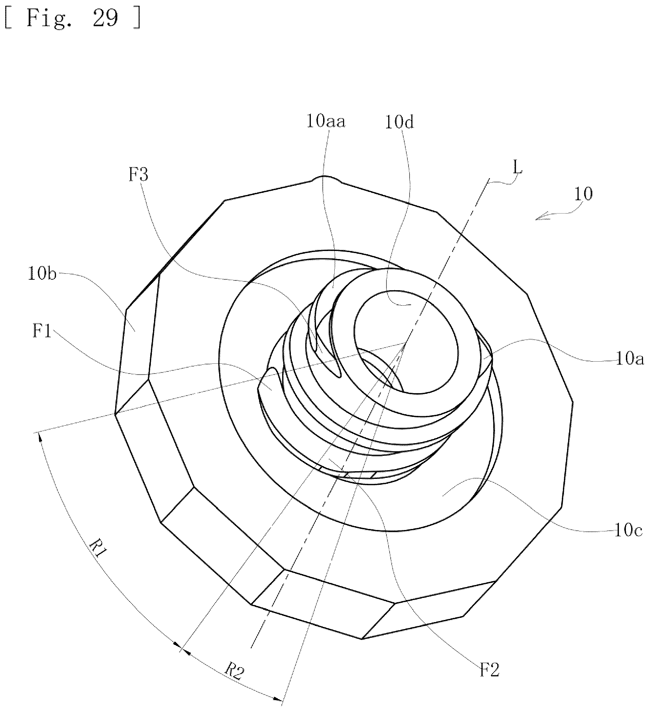

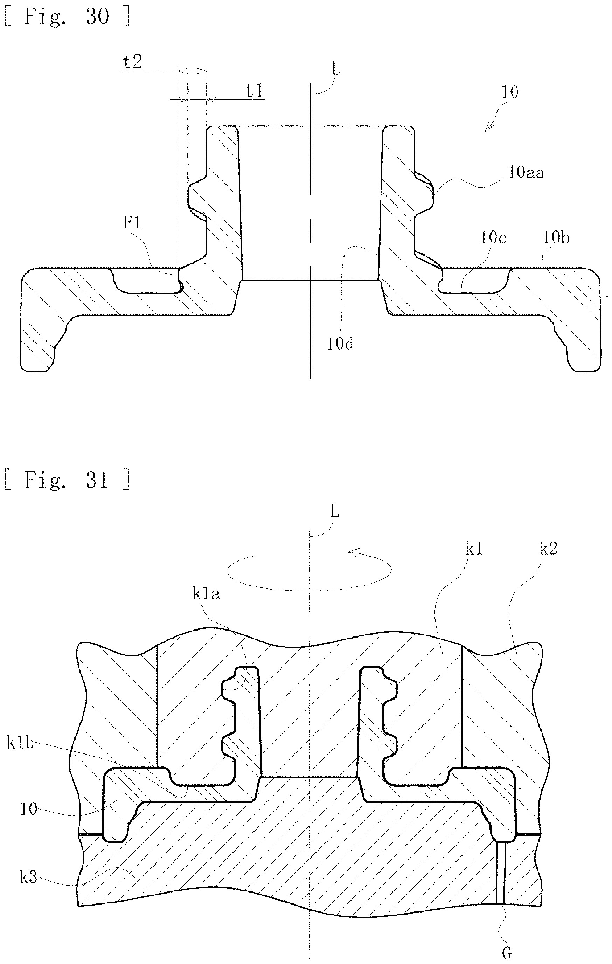

In particular, as illustrated in Figs. 29 and 30, in the thread shape 10aa of the locking part 10a according to the present embodiment, thread height t2 of the terminal end F1 is higher than thread height t1 in other portions. The thread shape 10aa of the locking part 10a according to the present embodiment includes a first thread region R1 that constitutes the terminal end F1, and a second thread region R2 whose height gradually increases toward the terminal end F1.

Specifically, as illustrated in Fig. 29, the thread shape 10aa is formed to spirally extend from the start end F3 on the tip end side of the locking part 10a to the terminal end F1 on the base end side, and includes the first thread region R1 which is the range where the thread height t1 is set to be highest at the terminal end F1, and the second thread region R2 which is the range whose height gradually (continuously) increases toward the terminal end F1. Note that the second thread region R2 is an expanded diameter part F2 where the thread shape gradually (continuously) increases in diameter.

In the present embodiment, the first thread region R1 (range where the terminal end F1 is formed) is formed over a range of 71° with respect to the central axis L of the cap part 10, and the second thread region R2 (range where the expanded diameter part F2 is formed) is formed over a range of 22° with respect to the central axis L of the cap part 10. The outer peripheral edge 10b of the cap part 10 has a large thickness and a polygonal shape in an outer peripheral direction.

The cap part 10 according to the present embodiment is comprised of a component obtained by resin molding, and as illustrated in Fig. 31, is produced using a rotating mold k1, and molds (k2, k3). The rotating mold k1 has, on its inner peripheral surface, a profile k1a corresponding to the thread shape 10aa of the locking part 10a, and at its outer peripheral end, a profile klb corresponding to the inner peripheral depression 10c, and is composed of a mold removable by rotation along the spiral of the thread shape 10aa and configured to mold the cap part 10 in collaboration with other molds k2, k3.

Specifically, the profiles k1a, kib of the cap part 10 are formed by the rotating mold k1 and other molds k2, k3, and resin is passed through gate G formed in the mold k3 into the profiles, and cooled and solidified. Subsequently, the rotating mold k1 is rotated around the central axis L, and moved in a direction away from the mold k3, thus can be removed while avoiding interference between the profile k1a of the rotating mold k1 and the formed thread shape 10aa of the locking part 10a.

The molds k2, k3 are each removed, thereby making it possible to obtain the cap part 10 having the thread shape 10aa corresponding to the profile k1a and the inner peripheral depression 10c corresponding to the profile klb. In the thread shape 10aa according to the present embodiment, the terminal end F1 has a higher thread height than that of other portions, thus when the rotating mold k1 is removed, the interference between the profile k1a of the rotating mold k1 and the formed thread shape 10aa of the locking part 10a can be easily and reliably avoided.

The access port member 7 according to the present embodiment includes: the body 8 having the opening 8c; the valve 9 made of an elastic member covering the opening 8c and attached to the attachment part 8d of the body 8, the valve 9 including the slit 9a that makes a transition from a closed state to an open state when pressed by the tip 11a of the syringe 11 (connecting member) capable of collecting blood or injecting a drug; and the cap part 10 fixed to the body 8 with the valve 9 interposed therebetween and the slit 9a facing outward, the cap part 10 having the locking part 10a with the thread shape 10aa to be screwed and locked into the thread shape 11b formed in the syringe 11 (connecting member). The thread shape 10aa of the locking part 10a is formed such that the thread height t2 of the terminal end F1 is higher than the thread height t1 in other portions, thus the lock work by the locking part 10a can be smoothly performed, and locking by the locking part can be securely and reliably conducted.

Specifically, the thread shape 10aa of the locking part 10a is formed such that the thread height t2 of the terminal end F1 is higher than the thread height t1 in other portions, thus as compared to when the thread is made higher over the entire thread shape 10aa or the thread is made higher in the range other than the terminal end F1, a large force is not required for screwing, therefore, the lock work by the locking part 10a can be smoothly performed, and in the process (the last stage) of a connection work made by screwing the syringe 11 into the thread shape 10aa, the terminal end F1 and the thread shape 11b of the syringe 11 can be elastically deformed, thus locking by the locking part can be securely and reliably conducted.

In particular, the body 8 is connected to a predetermined portion of the blood circuit for causing a patient's blood to extracorporeally circulate, thus the following effects can be achieved. Specifically, occurrence of a stagnation point on the rear surface of the valve 9 may cause blood clotting, thus such an occurrence needs to be reduced as much as possible. However, when the valve 9 is located closer to the flow route to prevent stagnation, the valve 9 cannot be sufficiently pressed by the tip 11a of the syringe 11, which makes it difficult to open the slit 9a. Thus, providing the valve 9 with the rib 9b as in the present embodiment allows the valve 9 to be sufficiently pressed by the tip 11a of the syringe 11, whereby both easiness of opening the slit 9a and less occurrence of a stagnation point can be achieved. In addition, providing the cap part 10 with the inner peripheral depression 10c can keep the height of the cap part 10 low, thereby making it possible for the tip 11a of the syringe 11 to sufficiently press the rib 9b of the valve 9.

The access port member 7 according to the present embodiment includes: the body 8 having the opening 8c; the valve 9 made of an elastic member covering the opening 8c and attached to the attachment part 8d of the body 8, the valve 9 including the slit 9a that makes a transition from a closed state to an open state when pressed by the tip 11a of the syringe 11 (connecting member) capable of collecting blood or injecting a drug; the cap part 10 fixed to the body 8 with the valve 9 interposed therebetween and the slit 9a facing outward; and the rib 9b as a seal part formed over the periphery of the slit 9a in the valve 9 and configured to, when the slit 9a is in an open state, match and seal the tip 11a of the syringe 11 (connecting member), thus when the slit 9a of the valve 9 is in an open state, leakage of liquid to the outside can be avoided by securing the sealing between the slit 9a and the syringe 11.

The rib 9b as a seal part maintains a contact state with the tip 11a of the syringe 11 (connecting member) during a transition of the slit 9a from a closed state to an open state and during a transition of the slit 9a from an open state to a closed state, thus in both cases where the syringe 11 is brought into contact with the front surface of the valve 9 to open the slit 9a and where the syringe 11 is moved away from the front surface of the valve 9 to close the slit 9a, leakage of liquid to the outside can be avoided by securing the sealing between the slit 9a and the syringe 11.

The seal part is comprised of the rib 9b which is formed by copying the shape of the tip 11a of the syringe 11 (connecting member), thus can avoid leakage of liquid to the outside by securing the sealing between the slit 9a and the syringe 11, and can facilitate connection work of the syringe 11 (connecting member) while preventing a failure in closing of the slit 9a. In addition, in the process (the last stage) of a connection work made by screwing the syringe 11 into the thread shape 10aa, locking by the locking part can be securely and reliably conducted by elastically deforming the terminal end F1 and the thread shape 11b of the syringe 11, therefore the state of contact between the rib 9b as the seal part and the tip 11a of the syringe 11 can be reliably achieved, and sealed state can be reliably maintained.

The thread shape 10aa of the locking part 10a includes the first thread region R1 that constitutes the terminal end F1, and the second thread region R2 whose height gradually increases toward the terminal end F1, thus in the process of a connection work made by screwing the syringe 11 into the thread shape 10aa, elastic deformation of the thread gradually increases in the second thread region R2 (expanded diameter part F2), and the maximum elastic deformation of the thread is achieved in the first thread region R1 (terminal end F1}, thus smooth screwing and lock work can be performed.

In addition, the first thread region R1 is formed over a range of approximately 70° with respect to the central axis L of the cap part 10, and the second thread region R2 is formed over a range of approximately 20° with respect to the central axis L of the cap part 10, thus smooth screwing and lock work can be performed in an appropriate range. The range of the first thread region R1 and the second thread region R2 can be set to any other angle.

However, in the cap part 10, the inner peripheral depression 10c is formed around the locking part 10a, and the outer peripheral edge 10b located on an outer diameter side of the inner peripheral depression 10c is formed to have a large thickness, thus the height dimension h (see Fig. 5) of the cap part 10 can be kept low, and upon connection of the syringe 11 as illustrated in Fig. 13, the tip surface 11c of the thread of the syringe 11 can be fitted into the inner peripheral depression 10c of the cap part 10, thus the tip 11a of the syringe 11 can be made further closer to the rib 9b of the valve 9. In this manner, the slit 9a can be opened sufficiently with the tip 11a of the syringe 11 by bringing the tip surface of the cap part 10 closer to the rib 9b of the valve 9. Therefore, the height dimension h of the locking part 10a in the cap part 10 can be kept low by providing the inner peripheral depression 10c, and upon connection of the syringe 11 as illustrated in Fig. 13, the tip surface 11c of the thread of the syringe 11 can be fitted into the inner peripheral depression 10c of the cap part 10, thus the tip 11a of the syringe 11 can be made further closer to the rib 9b of the valve 9, thereby making it possible for the tip 11a of the syringe 11 to sufficiently press the rib 9b of the valve 9.

The cap part 10 according to the present embodiment is comprised of a component obtained by resin molding, and produced using the rotating mold k1 having profile k1a on its inner peripheral surface and removable by rotation along the spiral of the thread shape 10aa, the profile k1a corresponding to the thread shape 10aa of the locking part 10a, thus when the rotating mold k1 is removed, the interference between the profile k1a of the rotating mold k1 and the formed thread shape 10aa of the locking part 10a can be easily and reliably avoided.

In the cap part 10 according to the present embodiment, the inner peripheral depression 10c is formed around the locking part 10a, and the outer peripheral edge 10b located on an outer diameter side of the inner peripheral depression 10c is formed to have a large thickness, and the rotating mold k1 has, at its outer peripheral end, the profile k1b corresponding to the inner peripheral depression 10c, thus the inner peripheral depression 10c along with the thread shape 10aa of the locking part 10a can be easily and smoothly formed.

In addition, the access port member 7 according to the present embodiment includes: the body 8 to be connected to a predetermined portion of a blood circuit for causing a patient's blood to extracorporeally circulate, the body 8 internally including the flow path 8b for circulating the blood and having the opening 8c communicating with the flow path 8b; the valve 9 made of an elastic member covering the opening 8c and attached to the attachment part 8d of the body 8, the valve 9 including the slit 9a that makes a transition from a closed state to an open state when pressed by the tip 11a of the syringe 11 (connecting member) capable of collecting blood or injecting a drug; the cap part 10 fixed to the body 8 with the valve 9 interposed therebetween and the slit 9a facing outward; and the rib 9b integrally formed over the periphery of the slit 9a in the valve 9, thus the access port member 7 can facilitate connection work of the syringe 11 (connecting member) while preventing a failure in closing of the slit 9a.

In addition, the cap part 10 according to the present embodiment includes the locking part 10a with the thread shape 10aa to be screwed and locked into the thread shape 11b of the syringe 11 (connecting member), thus the following effects can be achieved. Specifically, since the dimensions of the locking part 10a are strictly defined by a standard, the slit 9a of the valve 9 may not be sufficiently pressed by the tip 11a of the syringe 11 (connecting member), thus the slit 9a may not be sufficiently opened. However, when the valve 9 is moved to the side where the syringe 11 is connected to prevent such a failure (for example, when the front surface of the valve 9 is superficialized to the side where the syringe 11 is connected), the valve 9 is made thicker, making it further difficult to open the slit 9a. When the valve 9 is made thinner and the valve 9 is superficialized, a liquid stagnation point occurs in greater number on the flow route side of the valve 9, and stagnation of blood and liquid drug is likely to occur, and in particular, when blood stagnates, blood clotting may occur. Thus, a structure needs to be created in which the slit 9a is reliably opened and stagnation is unlikely to occur. Thus, according to the present embodiment, providing the valve 9 with the rib 9b allows the tip 11a of the syringe 11 to sufficiently press the valve 9, whereby both easiness of opening the slit 9a and prevention of a stagnation point can be achieved.

Although the present embodiment has been described above, the present invention is not limited to this, and the connecting member to be used is not limited to the syringe 11, and may be another connecting member (for example, a connecting member attached to the distal end of an infusion tube) which has a tip capable of pressing the rib 9b, and causes a transition of the slit 9a from a closed state to an open state by pressing to enable blood collection or drug injection. The depressions 8e may not be formed in the body 8. The valve 9 is not limited to the one having the contour of a rectangle in a plan view, and may be square, circular, elliptical or rectangular in a plan view. In addition, the present invention is not limited to the one connected to a flow route for blood in the blood circuit, and may be connected to various flow routes (such as a liquid drug infusion line serving as a flow route for a liquid drug) extending from the blood circuit.

In addition, the present invention is not limited to the one connected to a flow route for blood in the blood circuit, and may be connected to various flow routes (such as a liquid drug infusion line or a liquid drug delivery line serving as a flow route for a liquid drug) extending from the blood circuit. More specifically, in addition to the blood circuit for causing a patient's blood to extracorporeally circulate, the present invention is applicable to a circuit connected to a liquid delivery circuit, used for collection of blood flowing in the liquid delivery circuit, sampling of a liquid drug, and infusion of a liquid drug. For application to a liquid delivery circuit, an access port member may be used with one end thereof connected to an infusion bag and the other end connected to a connector coupled to an infusion line such as an indwelling needle or a blood circuit to enable drug injection and sampling, or an access port member may be used with connected to a liquid drug delivery circuit extended from a blood purifier such as the dialyzer 3 or various filters such as a blood adsorber, and a plasma separator to enable drug injection and sampling.

For application to a liquid delivery circuit, the access port member 12 having the configuration illustrated in Figs. 18 and 19 may be used. The access port member 12 includes: a body 13 having an opening 13b; a valve 9 made of an elastic member covering the opening 13b and attached to an attachment part of the body 13, the valve 9 including a slit 9a that makes a transition from a closed state to an open state when pressed by the tip 11a of the syringe 11 (connecting member) capable of collecting blood or injecting a drug; a cap part 10 fixed to the body 13 with the valve 9 interposed therebetween and the slit 9a facing outward, the cap part 10 having a locking part 10a with a thread shape 10aa to be screwed and locked into a thread shape 11b formed in the syringe 11; and a rib 9b integrally formed over the periphery of the slit 9a in the valve 9. The distal tip of a liquid delivery circuit H is to be connected to the connecting part 13a formed in the body 13. Note that the access port member 12 may be used instead of the connecting part K illustrated in Fig. 1.

In the first to third embodiments, the access port member is applied to a blood circuit in hemodialysis treatment, but may also be applied to other flow routes (such as a blood circuit installed in an apparatus used for e.g., hemodiafiltration, hemofiltration, AFBF, continuous renal replacement therapy, hemoadsorption, selective cytapheresis, simple plasma exchange, double filtration plasmapheresis, or plasma adsorption) for enabling purification treatment of a patient's blood.

A first aspect of the present invention provides an access port member including: a body 8 having an opening 8c; a valve 9 made of an elastic member covering the opening 8c and attached to an attachment part 8d of the body 8, the valve 9 including a slit 9a that makes a transition from a closed state to an open state when pressed by the tip 11a of a syringe 11 (connecting member) capable of collecting blood or injecting a drug; a cap part 10 fixed to the body 8 with the valve 9 interposed therebetween and the slit 9a facing outward, the cap part 10 having a locking part 10a with a thread shape 10aa to be screwed and locked into a thread shape 11b formed in the syringe 11 (connecting member); and a rib 9b integrally formed over the periphery of the slit 9a in the valve 9. Consequently, the effect of facilitating connection work of the syringe 11 (connecting member) while preventing a failure in closing of the slit 9a is achieved.

A second aspect of the present invention provides the access port member according to the first aspect, in which the rib 9b serves as a seal part that is formed over the periphery of the slit 9a in the valve 9 and configured to, when the slit 9a is in an open state, match and seal the tip 11a of the syringe 11 (connecting member). Thus, when the slit 9a of the valve 9 is in an open state, leakage of liquid to the outside can be avoided by securing the sealing between the slit 9a and the syringe 11.

A third aspect of the present invention provides the access port member according to the first aspect, in which the thread shape 10aa of the locking part 10a is formed such that the thread height t2 of the terminal end F1 is higher than the thread height t1 in other portions. Thus, the lock work by the locking part 10a can be smoothly performed, and locking by the locking part can be securely and reliably conducted.

A fourth aspect of the present invention provides the access port member according to the first aspect, in which the rib 9b serves as a seal part that is formed over the periphery of the slit 9a in the valve 9 and configured to, when the slit 9a is in an open state, match and seal the tip 11a of the syringe 11 (connecting member), and the thread shape 10aa of the locking part 10a is formed such that the thread height t2 of the terminal end F1 is higher than the thread height t1 in other portions. Thus, when the slit 9a of the valve 9 is in an open state, leakage of liquid to the outside can be avoided by securing the sealing between the slit 9a and the syringe 11, the lock work by the locking part 10a can be smoothly performed, and locking by the locking part can be securely and reliably conducted.

A fifth aspect of the present invention provides the access port member according to the first aspect, in which the rib 9b is composed of a protrusion formed on the front surface, the rear surface, or both surfaces of the valve 9, is circular, elliptical or rectangular in a plan view, and has an internal region 9bc in which the slit 9a is formed. Thus, elastic deformation is made easily by reducing an elastic force around the slit 9a so that an open state can be achieved, and a difference in level can be prevented from occurring by improving the restoring force after the elastic deformation with the rib 9b.

A sixth aspect of the present invention provides the access port member according to the fifth aspect, in which the slit 9a is formed in the internal region 9bc of the rib, and part of the slit 9a extends to the tip surface 9ba of the rib 9b. Thus, the slit 9a can be formed in the valve 9 without requiring a high positioning accuracy.

A seventh aspect of the present invention provides the access port member according to the first to sixth aspects, in which the slit 9a is formed in a line segment shape or a cross shape. Thus, an open state can be favorably achieved by pressing the tip 11a of the syringe 11.

An eighth aspect of the present invention provides an access port member including: a body 8 having an opening 8c; a valve 9 made of an elastic member covering the opening 8c and attached to an attachment part 8d of the body 8, the valve 9 including a slit 9a that makes a transition from a closed state to an open state when pressed by the tip 11a of a syringe 11 (connecting member) capable of collecting blood or injecting a drug; a cap part 10 fixed to the body 8 with the valve 9 interposed therebetween and the slit 9a facing outward, the cap part 10 having a locking part 10a with a thread shape 10aa to be screwed and locked into a thread shape 11b formed in the syringe 11 (connecting member); and a rib 9b as a seal part formed over the periphery of the slit 9a in the valve 9 and configured to, when the slit 9a is in an open state, match and seal the tip 11a of the syringe 11 (connecting member). Thus, when the slit 9a of the valve 9 is in an open state, leakage of liquid to the outside can be avoided by securing the sealing between the slit 9a and the syringe 11.

A ninth aspect of the present invention provides the access port member according to the eighth aspect, in which the thread shape 10aa of the locking part 10a is formed such that the thread height t2 of the terminal end F1 is higher than the thread height t1 in other portions. Thus, the lock work by the locking part 10a can be smoothly performed, and locking by the locking part can be securely and reliably conducted.

A tenth aspect of the present invention provides the access port member according to the eighth aspect, in which the rib 9b as a seal part maintains a contact state with the tip 11a of the syringe 11 (connecting member) during a transition of the slit 9a from a closed state to an open state and during a transition of the slit 9a from an open state to a closed state. Thus, in both cases where the syringe 11 is brought into contact with the front surface of the valve 9 to open the slit 9a and where the syringe 11 is moved away from the front surface of the valve 9 to close the slit 9a, leakage of liquid to the outside can be avoided by securing the sealing between the slit 9a and the syringe 11.

An eleventh aspect of the present invention provides the access port member according to the eighth aspect, in which the seal part is comprised of the rib 9b which is formed by copying the shape of the tip 11a of the syringe 11 (connecting member). Therefore, leakage of liquid to the outside can be avoided by securing the sealing between the slit 9a and the syringe 11, and connection work of the syringe 11 (connecting member) can be facilitated while preventing a failure in closing of the slit 9a.

A twelfth aspect of the present invention provides the access port member according to the eighth to eleventh aspects, in which the slit 9a is formed in a line segment shape or a cross shape. Thus, an open state can be favorably achieved by pressing the tip 11a of the syringe 11.

A thirteenth aspect of the present invention provides an access port member including: a body 8 having an opening 8c; a valve 9 made of an elastic member covering the opening 8c and attached to an attachment part 8d of the body 8, the valve 9 including a slit 9a that makes a transition from a closed state to an open state when pressed by the tip 11a of a syringe 11 (connecting member) capable of collecting blood or injecting a drug; and a cap part 10 fixed to the body 8 with the valve 9 interposed therebetween and the slit 9a facing outward, the cap part 10 having a locking part 10a with a thread shape 10aa to be screwed and locked into a thread shape 11b formed in the syringe 11 (connecting member). The thread shape 10aa of the locking part 10a is formed such that the thread height t2 of the terminal end F1 is higher than the thread height t1 in other portions. Thus, the lock work by the locking part 10a can be smoothly performed, and locking by the locking part can be securely and reliably conducted.

A fourteenth aspect of the present invention provides the access port member according to the thirteenth aspect, in which the thread shape 10aa of the locking part 10a includes a first thread region R1 that constitutes the terminal end F1, and a second thread region R2 where a thread height gradually increases toward the terminal end F1. Thus, in the process of a connection work made by screwing the syringe 11 into the thread shape 10aa, elastic deformation of the thread gradually increases in the second thread region R2 (expanded diameter part F2), and the maximum elastic deformation of the thread is achieved in the first thread region R1 (terminal end F1), thus smooth screwing and lock work can be performed.

A fifteenth aspect of the present invention provides the access port member according to the thirteenth or fourteenth aspect, in which in the cap part 10, the inner peripheral depression 10c is formed around the locking part 10a, and the outer peripheral edge 10b located on an outer diameter side of the inner peripheral depression 10c is formed to have a large thickness. Thus, the height dimension h of the cap part 10 can be kept low, and upon connection of the syringe 11, the tip surface 11c of the thread of the syringe 11 can be fitted into the inner peripheral depression 10c of the cap part 10, thus the tip 11a of the syringe 11 can be made further closer to the rib 9b of the valve 9.

A sixteenth aspect of the present invention provides a method of manufacturing the access port member according to the thirteenth to fifteenth aspects, in which the cap part 10 is comprised of a component obtained by resin molding, and produced using a rotating mold k1 having profile kla on its inner peripheral surface and removable by rotation along the spiral of the thread shape 10aa, the profile kla corresponding to the thread shape 10aa of the locking part 10a. Thus, when the rotating mold k1 is removed, the interference between the profile kla of the rotating mold k1 and the formed thread shape 10aa of the locking part 10a can be easily and reliably avoided.

A seventeenth aspect of the present invention provides a method of manufacturing the access port member according to the sixteenth aspect, in which in the cap part 10, the inner peripheral depression 10c is formed around the locking part 10a, and the outer peripheral edge 10b located on an outer diameter side of the inner peripheral depression 10c is formed to have a large thickness, and the rotating mold k1 has the profile klb corresponding to the inner peripheral depression 10c on an outer peripheral end. Thus, the inner peripheral depression 10c along with the thread shape 10aa of the locking part 10a can be easily and smoothly formed.

An eighteenth aspect of the present invention provides a flow route to be connected to the body 8 according to the first to seventeenth aspects, in which the flow route is comprised of a blood circuit configured to cause a patient's blood to extracorporeally circulate, a liquid delivery circuit configured to inject a liquid drug or blood to a patient, or a liquid drug delivery circuit configured to cause a liquid drug to flow. Consequently, the present invention is applicable to a blood circuit, a liquid delivery circuit configured to inject a liquid drug or blood to a patient, or a liquid drug delivery circuit configured to cause a liquid drug to flow.

### Industrial Applicability

The present invention is also applicable to any blood purification apparatus having a different appearance, additional functions, or the like within the spirit of the present invention.

### Reference Signs List

- 1: ARTERIAL BLOOD CIRCUIT
- 2: VENOUS BLOOD CIRCUIT
- 3: DIALYZER (BLOOD PURIFIER)
- 4: BLOOD PUMP
- 5: AIR-TRAP CHAMBER
- 6: DIALYSIS APPARATUS BODY
- 7: ACCESS PORT MEMBER
- 8: BODY
- 8a: CONNECTING PORTION
- 8b: FLOW PATH
- 8c: OPENING
- 8d: ATTACHMENT PART
- 8e: DEPRESSION
- 9: VALVE
- 9a: SLIT
- 9b: RIB
- 9ba: TIP SURFACE
- 9bb: LATERAL SURFACE
- 9bc: INTERNAL REGION
- 10: CAP PART
- 10a: LOCKING PART
- 10aa: THREAD SHAPE
- 10b: OUTER PERIPHERAL EDGE
- 10c: INNER PERIPHERAL DEPRESSION
- 10d: COMMUNICATION HOLE
- 11: SYRINGE (CONNECTING MEMBER)
- 11a: TIP
- 11b: THREAD SHAPE
- 12: ACCESS PORT MEMBER
- 13: BODY
- 13a: CONNECTING PART
- 13b: OPENING
- A: LONG SIDE
- B: SHORT SIDE
- La: LIQUID DRUG INFUSION LINE
- H: LIQUID DELIVERY CIRCUIT
- L: CENTRAL AXIS
- F1: TERMINAL END
- F2: EXPANDED DIAMETER PART
- F3: START END
- k1: ROTATING MOLD
- kla, klb: PROFILE
- k2, k3: MOLD
- G: GATE

## Claims

1. An access port member comprising:
a body having an opening;
a valve made of an elastic member covering the opening and attached to an attachment part of the body, the valve including a slit that makes a transition from a closed state to an open state when pressed by a tip of a connecting member capable of collecting blood or injecting a drug;
a cap part fixed to the body with the valve interposed therebetween and the slit facing outward, the cap part having a locking part with a thread shape to be screwed and locked into a thread shape formed in the connecting member; and
a rib integrally formed over a periphery of the slit in the valve.

2. The access port member according to Claim 1, further comprising a seal part formed over the periphery of the slit in the valve and configured to, when the slit is in an open state, match and seal the tip of the connecting member.

3. The access port member according to Claim 1,
wherein the thread shape of the locking part is formed such that a thread height at a terminal end is higher than a thread height in other portions.

4. The access port member according to Claim 1, further comprising a seal part formed over the periphery of the slit in the valve and configured to, when the slit is in an open state, match and seal the tip of the connecting member,
wherein the thread shape of the locking part is formed such that a thread height at a terminal end is higher than a thread height in other portions.

5. The access port member according to Claim 1,
wherein the rib is composed of a protrusion formed on a front surface, a rear surface, or both surfaces of the valve, is circular, elliptical or rectangular in a plan view, and has an internal region in which the slit is formed.

6. The access port member according to Claim 5,
wherein the slit is formed in the internal region of the rib, and part of the slit extends to a tip surface of the rib.

7. The access port member according to any one of Claims 1 to 6,
wherein the slit is formed in a line segment shape or a cross shape.

8. An access port member comprising:
a body having an opening;
a valve made of an elastic member covering the opening and attached to an attachment part of the body, the valve including a slit that makes a transition from a closed state to an open state when pressed by a tip of a connecting member capable of collecting blood or injecting a drug;
a cap part fixed to the body with the valve interposed therebetween and the slit facing outward, the cap part having a locking part with a thread shape to be screwed and locked into a thread shape formed in the connecting member; and
a seal part formed over a periphery of the slit in the valve and configured to, when the slit is in an open state, match and seal the tip of the connecting member.

9. The access port member according to Claim 8,
wherein the thread shape of the locking part is formed such that a thread height of a terminal end is higher than a thread height in other portions.

10. The access port member according to Claim 8,
wherein the seal part maintains a contact state with the tip of the connecting member during a transition of the slit from a closed state to an open state and during a transition of the slit from an open state to a closed state.

11. The access port member according to Claim 8,
wherein the seal part is comprised of a rib which is formed by copying a shape of the tip of the connecting member.

12. The access port member according to any one of Claims 8 to 11,
wherein the slit is formed in a line segment shape or a cross shape.

13. An access port member comprising:
a body having an opening;
a valve made of an elastic member covering the opening and attached to an attachment part of the body, the valve including a slit that makes a transition from a closed state to an open state when pressed by a tip of a connecting member capable of collecting blood or injecting a drug; and
a cap part fixed to the body with the valve interposed therebetween and the slit facing outward, the cap part having a locking part with a thread shape to be screwed and locked into a thread shape formed in the connecting member,
wherein the thread shape of the locking part is formed such that a thread height at a terminal end is higher than a thread height in other portions.

14. The access port member according to Claim 13,
wherein the thread shape of the locking part includes a first thread region that constitutes the terminal end, and a second thread region where a thread height gradually increases toward the terminal end.

15. The access port member according to Claim 13 or 14,
wherein in the cap part, an inner peripheral depression is formed around the locking part, and an outer peripheral edge located on an outer diameter side of the inner peripheral depression is formed to have a large thickness.

16. A method of manufacturing the access port member according to any one of Claims 13 to 15,
wherein the cap part is comprised of a component obtained by resin molding, and produced using a rotating mold having a profile on an inner peripheral surface and removable by rotation along a spiral of the thread shape, the profile corresponding to the thread shape of the locking part.

17. The method of manufacturing the access port member, according to Claim 16,
wherein in the cap part, an inner peripheral depression is formed around the locking part, an outer peripheral edge located on an outer diameter side of the inner peripheral depression is formed to have a large thickness, and the rotating mold has a profile corresponding to the inner peripheral depression on an outer peripheral end.

18. A flow route comprising: a blood circuit configured to cause a patient's blood to extracorporeally circulate; a liquid delivery circuit configured to inject a liquid drug or blood to a patient; or a liquid drug delivery circuit configured to cause a liquid drug to flow, the flow route being connected to the body according to any one of Claims 1 to 17.
